(19) Europäisches Patentamt
European Patent Office
Office européen des brevets

(11) EP 0 562 025 B1

(12) EUROPEAN PATENT SPECIFICATION

(45) Date of publication and mention of the grant of the patent: **07.02.2001 Bulletin 2001/06**

(21) Application number: **92903279.5**

(22) Date of filing: **20.11.1991**

(51) Int Cl.[7]: **A01N 1/02**, C12Q 1/00, G01N 33/566, G01N 33/543, B01J 19/00, C07D 471/02, C07D 235/00, C07D 473/00, C07D 235/30, C07K 1/04

(86) International application number: **PCT/US91/08693**

(87) International publication number: **WO 92/10092 (25.06.1992 Gazette 1992/14)**

(54) **Compounds and their use in a binary synthesis strategy**

Verbindungen und ihre Verwendung in einer binären Synthesestrategie

Substances et leur utilisation pour un strategie binaire de la synthése

(84) Designated Contracting States: **AT BE CH DE DK ES FR GB GR IT LI LU NL SE**

(30) Priority: **06.12.1990 US 624120**

(43) Date of publication of application: **29.09.1993 Bulletin 1993/39**

(60) Divisional application: **00202667.2 / 1 046 421**

(73) Proprietor: **Affymetrix, Inc. (a Delaware Corporation)**
**Santa Clara, CA 95051 (US)**

(72) Inventors:
- **FODOR, Stephen, P., A.** Palo Alto, CA 94303 (US)
- **STRYER, Lubert** Stanford, CA 94305 (US)
- **WINKLER, James, L.** Palo Alto, CA 94306 (US)
- **HOLMES, Christopher, W.** Sunnyvale, CA 94086 (US)
- **SOLAS, Dennis, W.** San Francisco, CA 94131 (US)

(74) Representative: **Bizley, Richard Edward et al**
**Hepworth, Lawrence, Bryer & Bizley**
**Merlin House**
**Falconry Court**
**Baker's Lane**
**Epping Essex CM16 5DQ (GB)**

(56) References cited:

| | |
|---|---|
| EP-A- 0 228 310 | WO-A-84/03564 |
| WO-A-90/04652 | WO-A-90/15070 |
| WO-A-91/07087 | US-A- 3 849 137 |
| US-A- 4 269 933 | US-A- 4 517 338 |
| US-A- 4 562 157 | US-A- 4 631 211 |
| US-A- 4 762 881 | |

- **PATENT ABSTRACTS OF JAPAN vol. 13, no. 557 (P-974), 12 December 1989 & JP 01 233447 A (OKI ELECTRIC IND. CO. LTD.), 19 September 1989 & DATABASE WPI Section Ch, Week 8943 Derwent Publications Ltd., London, GB; Class A08, AN 89-314201 & JP 01 233447 A (OKI ELECTRIC IND. K.K.) , 19 September 1989**
- **E. REICHMANIS ET AL.: "o-Nitrobenzyl Photochemistry: Solution vs. Solid-State Behaviour" JOURNAL OF POLYMER SCIENCE, POLYMER CHEMISTRY EDITION, vol. 23, no. 1, January 1985, pages 1-8, XP002105962 NEW YORK US**
- **D. McGILLIS, "Lithography", in VLSI Technology, published 1983 by McGraw-Hill Book Company (New York), pages 267-301, see pages 267-274.**
- **Science, Vol. 251, issued 15 February 1991, S. FODOR et al., "Light-Directed, Spatially Addressable Parallel Chemical Synthesis", pages 767-773, see entire document.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 562 025 B1

- CHEMICAL ABSTRACTS, Vol. 114, No. 15, issued 15 April 1991 (Columbus, Ohio, USA), S. ROBERTSON et al., "A general and efficient route for chemical aminoacylation of transfer RNAs", see page 839, cols. 1-2, the Abstract No. 143954x, J. Am. Chem. Soc. 1991, 113(7), 2722-9 (Eng), see entire Abstract.
- CHEMICAL ABSTRACTS, Vol. 112, No. 11, issued 12 March 1990 (Columbus, Ohio, USA), C. SHIN et al., "Dehydrooligopeptides. XI. Facile synthesis of various kinds of dehydrodi- and tripeptides, and dehydroenkephalins containing Tyr residue by using N-carboxydehydrotyrosine anhydride", see page 818, col.2, the Abstract No. 99207p, Bull. Chem. Soc. Jpn. 1989, 62(4), 1127-35 (Eng) see entire Abstract.
- Proceedings of the Indian National Science Academy, Vol. 53, No. 6, issued 1987, V.K. HARIDASAN et al, "Peptide Synthesis Using Photolytically Cleavalable 2-Nitro-benzyloxycarbonyl protecting group", pages 717-728, see Abstract; introduction, cpd (4); and page 727, lines 17-34.
- Applied Physics Letters, Vol. 31, No. 7, issued 01 October 1977, D. FLANDERS et al., "A new interferometric alignment technique", pages 426-429, see entire document.
- CHEMICAL ABSTRACTS, Vol. 114, No. 23, issued 10 June 1991 (Columbus, Ohio, USA), M. IWAMURA et al., "1-x-Diazobenzyl) pyrene: a reagent for photloabile and fluorescent protection of carboxyl groups of amino acids and peptides", see page 827, col. 1, the Abstract No. 229349r, Synlett 1991, (1), 35-6 (Eng), see entire Abstract.
- Journal of the American Society, Vol. 92, No. 21, issued 21 October 1970, A. PATCHORNIK et al., "Photosensitive Protecting Groups", pages 6333-6335. see page 6334, Scheme I.

(2) INFORMATION FOR SEQ ID NO:1:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:1:

```
Tyr Gly Gly Phe Leu
1               5
```

(2) INFORMATION FOR SEQ ID NO:2:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:2:

```
Pro Gly Gly Phe Leu
1               5
```

(2) INFORMATION FOR SEQ ID NO:3:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:3:

```
Tyr Gly Ala Gly Phe
1               5
```

(2) INFORMATION FOR SEQ ID NO:4:

(i) SEQUENCE CHARACTERISTICS:

(A) LENGTH: 6 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:4:

```
Tyr Gly Ala Phe Leu Ser
1               5
```

(2) INFORMATION FOR SEQ ID NO:5:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:5:

```
Tyr Gly Ala Phe Ser
1               5
```

(2) INFORMATION FOR SEQ ID NO:6:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:6:

```
Tyr Gly Ala Phe Leu
1               5
```

(2) INFORMATION FOR SEQ ID NO:7:

(i) SEQUENCE CHARACTERISTICS:

(A) LENGTH: 6 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:7:

```
Tyr Gly Gly Phe Leu Ser
1               5
```

(2) INFORMATION FOR SEQ ID NO:8:

(i) SEQUENCE CHARACTERISTICS:

(A) LENGTH: 4 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:8:

```
Tyr Gly Ala Phe
1
```

(2) INFORMATION FOR SEQ ID NO:9:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:9:

```
Tyr Gly Ala Leu Ser
1               5
```

(2) INFORMATION FOR SEQ ID NO:10:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:10:

```
Tyr Gly Gly Phe Ser
1               5
```

(2) INFORMATION FOR SEQ ID NO:11:

(i) SEQUENCE CHARACTERISTICS:

(A) LENGTH: 4 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:11:

```
Tyr Gly Ala Leu
1
```

(2) INFORMATION FOR SEQ ID NO:12:

(i) SEQUENCE CHARACTERISTICS:

(A) LENGTH: 6 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:12:

```
Tyr Gly Ala Phe Leu Phe
1               5
```

(2) INFORMATION FOR SEQ ID NO:13:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:13:

```
Tyr Gly Ala Phe Phe
1               5
```

(2) INFORMATION FOR SEQ ID NO:14:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:14:

```
Tyr Gly Gly Leu Ser
1               5
```

(2) INFORMATION FOR SEQ ID NO:15:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:15:

```
Tyr Gly Gly Phe Leu
1               5
```

(2) INFORMATION FOR SEQ ID NO:16:

(i) SEQUENCE CHARACTERISTICS:

(A) LENGTH: 6 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:16:

```
Tyr Gly Ala Phe Ser Phe
1               5
```

(2) INFORMATION FOR SEQ ID NO:17:

(i) SEQUENCE CHARACTERISTICS:

(A) LENGTH: 7 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:17:

```
Tyr Gly Ala Phe Leu Ser Phe
1               5
```

(2) INFORMATION FOR SEQ ID NO:18:

(i) SEQUENCE CHARACTERISTICS:

(A) LENGTH: 6 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:18:

```
Tyr Gly Ala Phe Met Gln
1               5
```

(2) INFORMATION FOR SEQ ID NO:19:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:19:

```
Tyr Gly Ala Phe Met
1               5
```

(2) INFORMATION FOR SEQ ID NO:20:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:20:

```
Tyr Gly Ala Phe Gln
1               5
```

(2) INFORMATION FOR SEQ ID NO:21:

(i) SEQUENCE CHARACTERISTICS:

(A) LENGTH: 5 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:21:

```
Tyr Gly Gly Phe Met
1               5
```

## Description

The present invention relates to the field of polymer synthesis. More specifically, the invention relates to compounds which provide protecting groups in an ordered method for forming a plurality of polymer sequences by sequential addition of reagents comprising the step of serially protecting and deprotecting portions of said plurality of polymer sequences. The invention also relates to light directed synthesis of diverse polymer sequences on substrates.

US-A-4,296,933 (Du Pont) describes a photopolymerizable coating composition comprising a non-gaseous, ethylenically unsaturated, polymerizable compound, a specified nitroaromatic compound, and an organic, radiation-sensitive, free-radical generating system useful for making a positive or negative polymeric image on a substrate.

In first aspect the invention provides a compound having the formula:

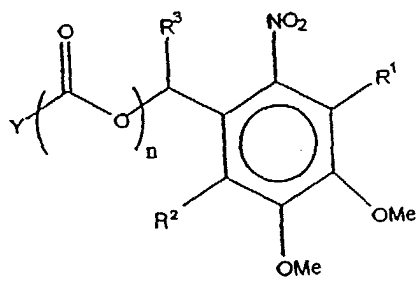

FORMULA 1

wherein n = 0 or 1; Y is a functional group; $R^1$ and $R^2$ independently are a hydrogen atom, an aryl, benzyl, halogen, hydroxyl, alkoxyl, thiol, thioether, amino, nitro, carboxyl, formate, formamido, sulfido, or phosphido group; and $R^3$ is a alkoxy, alkyl, aryl, or alkenyl group. Y may be selected from the group consisting of an oxygen of the carboxyl group of a natural or unnatural amino acid, an amino group of a natural or unnatural amino acid, or the C-5' oxygen group of a natural or unnatural deoxyribonucleic or ribonucleic acid.

In second aspect the invention provides a compound having the formula:

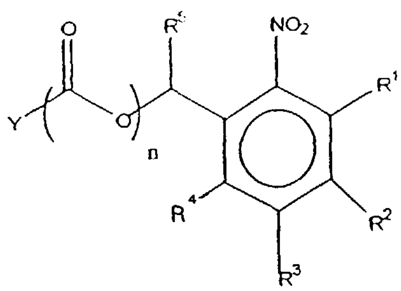

FORMULA 2

wherein n = 0 or 1; Y is a C-5' oxygen group of a natural or unnatural deoxyribonucleic and ribonucleic acid; $R^1$ through $R^4$ independently are a hydrogen atom, methyl ($R^3$ only), an aryl, benzyl, halogen, hydroxyl, alkoxyl, thiol, thioether, amino, nitro, carboxyl, formate, formamido, sulfido, or phosphido group; and $R^5$ is an alkoxy, alkyl, aryl, or alkenyl group; provided that (i) $R^2$ and $R^3$ are not both methoxy when $R^1$ and $R^4$ are hydrogen and $R^5$ is 2-nitrophenyl or 3, 4-dimethoxy-6 nitrophenyl, and (ii) $R^1$, $R^2$, $R^3$ and $R^4$ are not hydrogen simultaneously.

Preferably, in the compound of formula 2, $R^1$ and $R^4$ are selected independently from methoxy, hydrogen, alkoxy, and halogen.

In third aspect the invention provides a compound having the formula

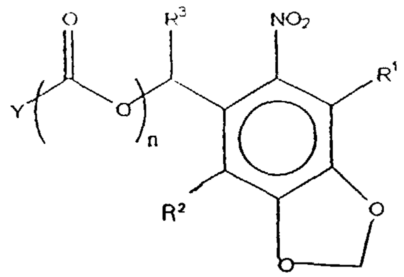

FORMULA 3

wherein n = 0 or 1; Y is a functional group; $R^1$ and $R^2$ independently are a hydrogen atom, an aryl, benzyl, halogen, hydroxyl, alkoxyl, thiol, thioether, amino, nitro, carboxyl, formate, formamido, sulfido, or phosphido group; and $R^3$ is alkoxy, alkyl, aryl, or alkenyl.

In the compound of Formula 1 or Formula 3, $R^1$ and $R^2$ are preferably chosen independently from methoxy, hydrogen, alkoxy, and halogen.

In the case of a compound of Formula 2 $R^5$ may be methyl whereas in the case of any other compound $R^3$ may be methyl.

In any of the compounds of formulae 1, 2 or 3, Y may be a functional group of a molecule selected from natural and unnatural amino acids and peptides, nucleosides, and oligonucleotides.

Advantageously, the compounds of the invention provide improved photoremovable protecting groups.

In fourth aspect the invention provides the use of a compound of Formula 1 or Formula 3 to provide protecting groups in an ordered method for forming a plurality of polymer sequences by sequential addition of reagents comprising the step of serially protecting and deprotecting portions of said plurality of polymer sequences for addition of other portions of said polymer sequences using a binary synthesis strategy. This aspect of the invention provides improved masking techniques for VSLIPS.

Preferably, said binary synthesis strategy is

(a) a binary masking strategy, and wherein said masking strategy provides at least two consecutive steps in which a mask factors a previous mask by protecting a portion of a previously illuminated portion from light and exposing a portion of a previously protected portion to light; or

(b) a binary masking strategy and wherein said masks are arranged in a gray code masking strategy, said gray code masking strategy having one edge illumination on each of a plurality of synthesis sites.

The use of the compounds may further comprise the step of forming a portion of said polymers with a non-binary masking strategy.

The compounds of the invention can be used in a reactor system for synthesis of diverse polymer sequences on a substrate. Such a reactor system provides for contacting reaction fluids to a substrate and delivering selected reaction fluids to the reactor. There is also a translation stage for moving a mask or substrate from at least a first relative location relative to a second relative location; a light for illuminating the substrate through a mask at selected times; and an appropriately programmed digital computer for selectively directing a flow of fluids from the reactor system, selectively activating the translation stage, and selectively illuminating the substrate so as to form a plurality of diverse polymer sequences on the substrate at predetermined locations.

The compounds of the invention can be used in a technique for selection of linker molecules in VLSLIPS, also a method of screening a plurality of linker polymers for use in binding affinity studies. In forming a plurality of linker polymers on a substrate in selected regions, the linker polymers are formed by the steps of recursively: on a surface of a substrate, irradiating a portion of the selected regions to remove a protecting group, and contacting the surface with a monomer; contacting the plurality of linker polymers with a ligand; and contacting the ligand with a labeled receptor.

The invention permits improved masking techniques for VLSLIPS. In one aspect of the masking technique, the invention permits an ordered method for forming a plurality of polymer sequences by sequential addition of reagents comprising the step of serially protecting and deprotecting portions of the plurality of polymer sequences for addition of other portions of the polymer sequences using a binary synthesis strategy.

Improved data collection equipment and techniques can be employed. For example, the instrumentation may include a system for determining affinity of a receptor to a ligand comprising: means for applying light to a surface of a substrate, the substrate comprising a plurality of ligands at predetermined locations, the means for applying directing light providing simultaneous illumination at a plurality of the predetermined locations; and an array of detectors for

detecting fluorescence at the plurality of predetermined locations. The invention also permits improved data analysis techniques including the steps of exposing fluorescently labelled receptors to a substrate, the substrate comprising a plurality of ligands in regions at known locations; at a plurality of data collection points within each of the regions, determining an amount of fluorescence from the data collection points; removing the data collection points deviating from a predetermined statistical distribution; and determining a relative binding affinity of the receptor from remaining data collection points.

Protected amino acid N-carboxy anhydrides for use in polymer synthesis are also permitted. For example, a compound having the following formula may be employed:

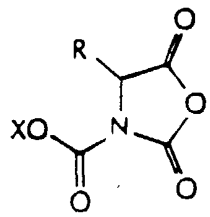

where R is a side chain of a natural or unnatural amino acid and X is a photoremovable protecting group.

A further understanding of the nature and advantages of the inventions herein may be realized by reference to the remaining portions of the specification and the attached drawings.

BRIEF DESCRIPTION OF THE DRAWINGS

Fig. 1 schematically illustrates light-directed spatially-addressable parallel chemical synthesis;
Fig. 2 schematically illustrates one example of light-directed peptide synthesis;
Fig. 3 schematically illustrates the software for the automated system for synthesizing diverse polymer sequences;
Fig. 4a and 4b illustrate operation of a program for polymer synthesis;
Fig. 5 is a schematic illustration of a "pure" binary masking strategy;
Fig. 6 is a schematic illustration of a gray code binary masking strategy;
Fig. 7 is a schematic illustration of a modified gray code binary masking strategy;
Fig. 8a schematically illustrates a masking strategy for a four step synthesis;
Fig. 8b schematically illustrates synthesis of all 400 peptide dimers;
Fig. 9 is a coordinate map for the ten-step binary synthesis;
Fig. 10 schematically illustrates a data collection system;
Fig. 11 is a block diagram illustrating the architecture of the data collection system;
Fig. 12 is a flow chart illustrating operation of software for the data collection/analysis system; and
Fig. 13 schematically illustrates one example of light-directed oligonucleotide synthesis.

DESCRIPTION OF THE PREFERRED EMBODIMENTS

CONTENTS

I. Definitions

II. General

- A. Deprotection and Addition
  1. Example
  2. Example
- B. Antibody recognition
  1. Example

III. Synthesis

A. Reactor System

B. Binary Synthesis Strategy

1. Example
2. Example
3. Example
4. Example
5. Example
6. Example

C. Linker Selection

D. Protecting Groups

1. Use of Photoremovable Protecting Groups During Solid-Phase Synthesis of Peptides
2. Use of Photoremovable Protecting Groups During Solid-Phase Synthesis of Oligonucleotides

E. Amino Acid N-Carboxy Anhydrides Protected with a Photoremovable Group

IV. Data Collection

A. Data Collection System

B. Data Analysis

V. Other Representative Applications
A. Oligonucleotide Synthesis
1. Example

VI. Conclusion

I. Definitions

Certain terms used herein are intended to have the following general definitions:

1. Complementary: Refers to the topological compatibility or matching together of interacting surfaces of a ligand molecule and its receptor. Thus, the receptor and its ligand can be described as complementary, and furthermore, the contact surface characteristics are complementary to each other.

2. Epitope: The portion of an antigen molecule which is delineated by the area of interaction with the subclass of receptors known as antibodies.

3. Ligand: A ligand is a molecule that is recognized by a particular receptor. Examples of ligands that can be investigated by this invention include, but are not restricted to, agonists and antagonists for cell membrane receptors, toxins and venoms, viral epitopes, hormones (e.g., opiates, steroids, etc.), hormone receptors, peptides, enzymes, enzyme substrates, cofactors, drugs, lectins, sugars, oligonucleotides, nucleic acids, oligosaccharides, proteins, and monoclonal antibodies.

4. Monomer: A member of the set of small molecules which can be joined together to form a polymer. The set of monomers includes but is not restricted to, for example, the set of common L-amino acids, the set of D-amino acids, the set of synthetic amino acids, the set of nucleotides and the set of pentoses and hexoses. As used herein, monomer refers to any member of a basis set for synthesis of a polymer. For example, dimers of the 20 naturally occurring L-amino acids form a basis set of 400 monomers for synthesis of polypeptides. Different basis sets of monomers may be used at successive steps in the synthesis of a polymer. Furthermore, each of the sets may include protected members which are modified after synthesis.

5. Peptide: A polymer in which the monomers are alpha amino acids and which are joined together through amide bonds and is alternatively referred to as a polypeptide. In the context of this specification it should be appreciated that the amino acids may be the L-optical isomer or the D-optical isomer. Peptides are often two or more amino acid monomers long, and often more than 20 amino acid monomers long. Standard abbreviations for amino acids are used (e.g., P for proline). These abbreviations are included in Stryer, Biochemistry, Third Ed., 1988,

6. Radiation: Energy which may be selectively applied including energy having a wavelength of between $10^{-14}$ and 104 meters including, for example, electron beam radiation, gamma radiation, x-ray radiation, ultraviolet radiation, visible light, infrared radiation, microwave radiation, and radio waves. "Irradiation" refers to the application of radiation to a surface.

7. Receptor: A molecule that has an affinity for a given ligand. Receptors may be naturally-occurring or synthetic molecules. Also, they can be employed in their unaltered state or as aggregates with other species. Receptors may be attached, covalently or noncovalently, to a binding member, either directly or via a specific binding substance. Examples of receptors which can be employed by this invention include, but are not restricted to, antibodies, cell membrane receptors, monoclonal antibodies and antisera reactive with specific antigenic determinants (such as on viruses, cells, or other materials), drugs, polynucleotides, nucleic acids, peptides, cofactors, lectins, sugars, polysaccharides, cells, cellular membranes, and organelles. Receptors are sometimes referred to in the art as anti-ligands. As the term receptors is used herein, no difference in meaning is intended. A "Ligand Receptor Pair" is formed when two macromolecules have combined through molecular recognition to form a complex.

Other examples of receptors which can be investigated by this invention include but are not restricted to:

a) Microorganism receptors: Determination of ligands which bind to receptors, such as specific transport proteins or enzymes essential to survival of microorganisms, is useful for a new class of antibiotics. Of particular value would be antibiotics against opportunistic fungi, protozoa, and those bacteria resistant to the antibiotics in current use.
b) Enzymes: For instance, determining the binding site of enzymes such as the enzymes responsible for cleaving neurotransmitters provides useful information. Determination of ligands which bind to certain receptors to modulate the action of the enzymes which cleave the different neurotransmitters is useful in the development of drugs which can be used in the treatment of disorders of neurotransmission.
c) Antibodies: For instance, the invention may be useful in investigating the ligand-binding site on the antibody molecule which combines with the epitope of an antigen of interest; determining a sequence that mimics an antigenic epitope may lead to the development of vaccines of which the immunogen is based on one or more of such sequences or lead to the development of related diagnostic agents or compounds useful in therapeutic treatments such as for autoimmune diseases (e.g., by blocking the binding of the "self" antibodies).
d) Nucleic Acids: Sequences of nucleic acids may be synthesized to establish DNA or RNA binding sequences.
e) Catalytic Polypeptides: Polymers, preferably polypeptides, which are capable of promoting a chemical reaction involving the conversion of one or more reactants to one or more products. Such polypeptides generally include a binding site specific for at least one reactant or reaction intermediate and an active functionality proximate to the binding site, in which the functionality is capable of chemically modifying the bound reactant. Catalytic polypeptides are described in, for example, U.S. application Serial No. 404,920.
f) Hormone receptors: For instance, the receptors for insulin and growth hormone. Determination of the ligands which bind with high affinity to a receptor is useful in the development of, for example, an oral replacement of the daily injections which diabetics must take to relieve the symptoms of diabetes, and in the other case, a replacement for the scarce human growth hormone which can only be obtained from cadavers or by recombinant DNA technology. Other examples are the vasoconstrictive hormone receptors; determination of those ligands which bind to a receptor may lead to the development of drugs to control blood pressure.
g) Opiate receptors: Determination of ligands which bind to the opiate receptors in the brain is useful in the development of less-addictive replacements for morphine and related drugs.

8. Substrate: A material having a rigid or semi-rigid surface. In many embodiments, at least one surface of the substrate will be substantially flat, although in some embodiments it may be desirable to physically separate synthesis regions for different polymers with, for example, wells, raised regions, etched trenches, or the like. According to other embodiments, small beads may be provided on the surface which may be released upon completion of the synthesis.

9. Protecting group: A material which is chemically bound to a monomer unit and which may be removed upon selective exposure to an activator such as electromagnetic radiation. Examples of protecting groups with utility

herein include those comprising nitropiperonyl, pyrenylmethoxy-carbonyl, nitroveratryl, nitrobenzyl, dimethyl dimethoxybenzyl, 5-bromo-7-nitroindolinyl, *o*-hydroxy-α-methyl cinnamoyl, and 2-oxymethylene anthraquinone.

10. Predefined Region: A predefined region is a localized area on a surface which is, was, or is intended to be activated for formation of a polymer. The predefined region may have any convenient shape, e.g., circular, rectangular, elliptical, wedge-shaped, etc. For the sake of brevity herein, "predefined regions" are sometimes referred to simply as "regions."

11. Substantially Pure: A polymer is considered to be "substantially pure" within a predefined region of a substrate when it exhibits characteristics that distinguish it from other predefined regions. Typically, purity will be measured in terms of biological activity or function as a result of uniform sequence. Such characteristics will typically be measured by way of binding with a selected ligand or receptor.

12. Activator refers to an energy source adapted to render a group active and which is directed from a source to a predefined location on a substrate. A primary illustration of an activator is light. Other examples of activators include ion beams, electric fields, magnetic fields, electron beams, x-ray, and the like.

13. Binary Synthesis Strategy refers to an ordered strategy for parallel synthesis of diverse polymer sequences by sequential addition of reagents which may be represented by a reactant matrix, and a switch matrix, the product of which is a product matrix. A reactant matrix is a 1 x m matrix of the building blocks to be added. The switch matrix is all or a subset of the binary numbers, preferably ordered, between 1 and m arranged in columns. In preferred embodiments, a binary strategy is one in which at least two successive steps illuminate half of a region of interest on the substrate. In most preferred embodiments, binary synthesis refers to a synthesis strategy which also factors a previous addition step. For example, a strategy in which a switch matrix for a masking strategy halves regions that were previously illuminated, illuminating about half of the previously illuminated region and protecting the remaining half (while also protecting about half of previously protected regions and illuminating about half of previously protected regions). It will be recognized that binary rounds may be interspersed with non-binary rounds and that only a portion of a substrate may be subjected to a binary scheme, but will still be considered to be a binary masking strategy within the definition herein. A binary "masking" strategy is a binary synthesis which uses light to remove protecting groups from materials for addition of other materials such as amino acids. In preferred embodiments, selected columns of the switch matrix are arranged in order of increasing binary numbers in the columns of the switch matrix.

14. Linker refers to a molecule or group of molecules attached to a substrate and spacing a synthesized polymer from the substrate for exposure/binding to a receptor.

II. General

Synthetic strategies and devices for the creation of large scale chemical diversity are described. For example, solid-phase chemistry, photolabile protecting groups, and photolithography are brought together to achieve light-directed spatially-addressable parallel chemical synthesis.
The preparation of peptides and nucleotides is described, but other polymers may also be prepared.
Such polymers include, for example, both linear and cyclic polymers of nucleic acids, polysaccharides, phospholipids, and peptides having either α-, β-, or ω-amino acids, heteropolymers in which a known drug is covalently bound to any of the above, polyurethanes, polyesters, polycarbonates, polyureas, polyamides, polyethyleneimines, polyarylene sulfides, polysiloxanes, polyimides, polyacetates, or other polymers which will be apparent upon review of this disclosure. It will be recognized further, that illustrations herein are primarily with reference to C- to N-terminal synthesis, but N- to C-terminal synthesis may also be performed.

A. Deprotection and Addition

A masked light source or other activator is used to direct the simultaneous synthesis of many different chemical compounds. Fig. 1 is a flow chart illustrating the process of forming chemical compounds according to one embodiment of the invention. Synthesis occurs on a solid support 2. A pattern of illumination through a mask 4a using a light source 6 determines which regions of the support are activated for chemical coupling. Activation may be accomplished by using light to remove photolabile protecting groups from selected areas of the substrate.
After deprotection, a first of a set of building blocks (indicated by "A" in Fig. 1), each bearing a photolabile protecting group (indicated by "X") is exposed to the surface of the substrate and it reacts with regions that were

addressed by light in the preceding step. The substrate is then illuminated through a second mask 4b, which activates another region for reaction with a second protected building block "B". The pattern of masks used in these illuminations and the sequence of reactants define the ultimate products and their locations, resulting in diverse sequences at predefined locations, as shown with the sequences ACEG and BDFH in the lower portion of Fig. 1. A large number of compounds may be formed in a small number of chemical steps by taking advantage of combinatorial masking strategies.

A high degree of miniaturization is possible because the density of compounds is determined largely with regard to spatial addressability of the activator, in one case the diffraction of light. Each compound is physically accessible and its position is precisely known. Hence, the array is spatially-addressable and its interactions with other molecules can be assessed.

The substrate may contain amino groups that are blocked with a photolabile protecting group as shown in Fig. 1. Amino acid sequences are made accessible for coupling to a receptor by removal of the photoprotecting groups.

When a polymer sequence to be synthesized is, for example, a polypeptide, amino groups at the ends of linkers attached to a glass substrate are derivatized with nitroveratryloxycarbonyl (NVOC), a photoremovable protecting group. The linker molecules may be, for example, aryl acetylene, ethylene glycol oligomers containing from 2-10 monomers, diamines, diacids, amino acids, or combinations thereof. Photodeprotection is effected by illumination of the substrate through, for example, a mask wherein the pattern has transparent regions with dimensions of, for example, less than 1 $cm^2$, $10^{-1}$ $cm^2$, $10^{-2}$ $cm^2$, $10^{-3}$ $cm^2$, $10^{-4}$ $cm^2$, $10^{-5}$ $cm^2$, $10^{-6}$ $cm^2$, $10^{-7}$ $cm^2$, $10^{-8}$ $cm^2$, or $10^{-10}$ $cm^2$. For example, the regions are between about 10x10 μm and 500x500 μm. The masks may be arranged to produce a checkerboard array of polymers, although any one of a variety of geometric configurations may be utilized.

1. Example

Free amino groups were fluorescently labelled by treatment of the entire substrate surface with fluorescein isothiocynate (FITC) after photodeprotection. Glass microscope slides were cleaned, aminated by treatment with 0.1% aminopropyltriethoxysilane in 95% ethanol, and incubated at 110°C for 20 min. The aminated surface of the slide was then exposed to a 30 mM solution of the N-hydroxysuccinimide ester of NVOC-GABA (nitroveratryloxycarbonyl-τ-amino butyric acid) in DMF. The NVOC protecting group was photolytically removed by imaging the 365 nm output from a Hg arc lamp through a chrome on glass 100 μm checkerboard mask onto the substrate for 20 min at a power density of 12 $mW/cm^2$. The exposed surface was then treated with 1 mM FITC in DMF. The substrate surface was scanned in an epi-fluorescence microscope (Zeiss Axioskop 20) using 488 nm excitation from an argon ion laser (Spectra-Physics model 2025). The fluorescence emission above 520 nm was detected by a cooled photomultiplier (Hamamatsu 943-02) operated in a photon counting mode. Fluorescence intensity was translated into a color display with red in the highest intensity and black in the lowest intensity areas. The presence of a high-contrast fluorescent checkerboard pattern of 100x100 μm elements revealed that free amino groups were generated in specific regions by spatially-localized photodeprotection.

2. Example

Fig. 2 is a flow chart illustrating this example . Carboxy-activated NVOC-leucine was allowed to react with an aminated substrate. The carboxy activated HOBT ester of leucine and other amino acids used in this synthesis was formed by mixing 0.25 mmol of the NVOC amino protected amino acid with 37 mg HOBT (1-hydroxybenzotriazole), 111 mg BOP (benzotriazolyl-n-oxy-tris (dimethylamino)-phosphoniumhexa-fluorophosphate) and 86 μl DIEA (diisopropylethylamine) in 2.5 ml DMF. The NVOC protecting group was removed by uniform illumination. Carboxy-activated NVOC-phenylalanine was coupled to the exposed amino groups for 2 hours at room temperature, and then washed with DMF and methylene chloride. Two unmasked cycles of photodeprotection and coupling with carboxy-activated NVOC-glycine were carried out. The surface was then illuminated through a chrome on glass 50 μm checkerboard pattern mask. Carboxy-activated *N*α-tBOC-*O*-tButyl-L-tyrosine was then added. The entire surface was uniformly illuminated to photolyze the remaining NVOC groups. Finally, carboxy-activated NVOC-L-proline was added, the NVOC group was removed by illumination, and the t-BOC and t-butyl protecting groups were removed with TFA. After removal of the protecting groups, the surface consisted of a 50 μm checkerboard array of Tyr-Gly-Gly-Phe-Leu (YGGFL) and Pro-Gly-Gly-Phe-Leu (PGGFL). See also SEQ ID NO:1 and SEQ ID NO:2.

B. Antibody Recognition

The substrate may be used to determine which of a plurality of amino acid sequences is recognized by an antibody of interest.

1. Example

In one example, the array of pentapeptides in the example illustrated in Fig. 2 was probed with a mouse monoclonal antibody directed against β-endorphin. This antibody (called 3E7) is known to bind YGGFL and YGGFM (see also SEQ ID NO:1 and SEQ ID NO:21) with nanomolar affinity and is discussed in Meo et al., Proc. Natl. Acad. Sci. USA (1983) 80:4084. This antibody requires the amino terminal tyrosine for high affinity binding. The array of peptides formed as described in Fig. 2 was incubated with a 2 μg/ml mouse monoclonal antibody (3E7) known to recognize YGGFL. See also SEQ ID NO:1. 3E7 does not bind PGGFL. See also SEQ ID NO:2. A second incubation with fluoresceinated goat anti-mouse antibody labeled the regions that bound 3E7. The surface was scanned with an epi-fluorescence microscope. The results showed alternating bright and dark 50 μm squares indicating that YGGFL (SEQ ID NO:1) and PGGFL (SEQ ID NO:2) were synthesized in a geometric array determined by the mask. A high contrast (>12:1 intensity ratio) fluorescence checkerboard image shows that (a) YGGFL (SEQ ID NO:1) and PGGFL (SEQ ID NO:2) were synthesized in alternate 50 μm squares, (b) YGGFL (SEQ ID NO:1) attached to the surface is accessible for binding to antibody 3E7, and (c) antibody 3E7 does not bind to PGGFL (SEQ ID NO:2)

A three-dimensional representation of the fluorescence intensity data in a 2 square by 4 square rectangular portion of the checkerboard was produced. It shows that the border between synthesis sites is sharp. The height of each spike in this display is linearly proportional to the integrated fluorescence intensity in a 2.5 μm pixel. The transition between PGGFL and YGGFL occurs within two spikes (5 μm). There is little variation in the fluorescence intensity of different YGGFL squares. The mean intensity of sixteen YGGFL synthesis sites was $2.03 \times 10^5$ counts and the standard deviation was $9.6 \times 10^3$ counts.

III. Synthesis

A. Reactor System

Fig. 3 schematically illustrates a device used to synthesize diverse polymer sequences on a substrate. The device includes an automated peptide synthesizer 401. The automated peptide synthesizer is a device which flows selected reagents through a flow cell 402 under the direction of a computer 404. The synthesizer may be an ABI Peptide Synthesizer, model no. 431A. The computer may be selected from a wide variety of computers or discrete logic including for, example, an IBM PC-AT or similar computer linked with appropriate internal control systems in the peptide synthesizer. The PC is provided with signals from the board computer indicative of, for example, the end of a coupling cycle.

Substrate 406 is mounted on the flow cell, forming a cavity between the substrate and the flow cell. Selected reagents flow through this cavity from the peptide synthesizer at selected times, forming an array of peptides on the face of the substrate in the cavity. Mounted above the substrate, and preferably in contact with the substrate is a mask 408. Mask 408 is transparent in selected regions to a selected wavelength of light and is opaque in other regions to the selected wavelength of light. The mask is illuminated with a light source 410 such as a UV light source. In one example the light source 410 is a model no. 82420 made by Oriel. The mask is held and translated by an x-y-z translation stage 412 such as an x-y translation stage made by Newport Corp. The computer coordinates action of the peptide synthesizer, x-y translation stage, and light source. Of course, the substrate may be translated instead of the mask.

In operation, the substrate is mounted on the flow cell. The substrate, with its surface protected by a suitable photo removable protecting group, is exposed to light at selected locations by positioning the mask and directing light from a light source, through the mask, onto the substrate for a desired period of time (such as, for example, 1 sec to 60 min in the case of peptide synthesis). A selected peptide or other monomer/polymer is pumped through the reactor cavity by the peptide synthesizer for binding at the selected locations on the substrate. After a selected reaction time (such as about 1 sec to 300 min in the case of peptide reactions) the monomer is washed from the system, the mask is appropriately repositioned or replaced, and the cycle is repeated.

Reactions may be conducted at or near ambient temperature.

Figs 4a and 4b are flow charts of the software used in operation of the reactor system. At step 502 the peptide synthesis software is initialized. At step 504 the system calibrates positioners on the x-y translation stage and begins a main loop. At step 506 the system determines which, if any, of the function keys on the computer have been pressed. If F1 has been pressed, the system prompts the user for input of a desired synthesis process. If the user enters F2, the system allows a user to edit a file for a synthesis process at step 510. If the user enters F3 the system loads a process from a disk at step 512. If the user enters F4 the system saves an entered or edited process to disk at step 514. If the user selects F5 the current process is displayed at step 516 while selection of F6 starts the main portion of the program, i.e., the actual synthesis according to the selected process. If the user selects F7 the system displays the location of the synthesized peptides, while pressing F10 returns the user to the disk operating system.

Fig. 4b illustrates the synthesis step 518 in greater detail. The main loop of the program is started in which the system first moves the mask to a next position at step 526. During the main loop of the program, necessary chemicals

flow through the reaction cell under the direction of the on-board computer in the peptide synthesizer. At step 528 the system then waits for an exposure command and, upon receipt of the exposure command exposes the substrate for a desired time at step 530. When an acknowledgement of complete exposure is received at step 532 the system determines if the process is complete at step 534 and, if so, waits for additional keyboard input at step 536 and, thereafter, exits the perform synthesis process.

A computer program used for operation of the system described above is written in Turbo C (Borland Int'l) and has been implemented in an IBM compatible system. The motor control software is adapted from software produced by Newport Corporation. It will be recognized that a large variety of programming languages could be utilized without departing from the scope of the invention herein. Certain calls are made to a graphics program in "Programmer Guide to PC and PS2 Video Systems" (Wilton, Microsoft Press, 1987).

Alignment of the mask may be achieved by one of two methods. Firstly, the system may rely upon relative alignment of the various components, which is normally acceptable since x-y-z translation stages are capable of sufficient accuracy for the purposes herein. Alternatively, alignment marks on the substrate are coupled to a CCD device for appropriate alignment.

Pure reagents need not necessarily be added at each step nor need complete photolysis of the protecting groups be provided at each step. Multiple products will thereby be formed in each synthesis site. For example, if the monomers A and B are mixed during a synthesis step, A and B will bind to deprotected regions, roughly in proportion to their concentration in solution. Hence, a mixture of compounds will be formed in a synthesis region. A substrate formed with mixtures of compounds in various synthesis regions may be used to perform, for example, an initial screening of a large number of compounds, after which a smaller number of compounds in regions which exhibit high binding affinity are further screened. Similar results may be obtained by only partially photolyzing a region, adding a first monomer, re-photolyzing the same region, and exposing the region to a second monomer.

B. <u>Binary Synthesis Strategy</u>

In a light-directed chemical synthesis, the products formed depend on the pattern and order of masks, and on the order of reactants. To make a set of products there will in general be a finite number of possible masking strategies. A binary synthesis strategy may be utilized.

The binary synthesis strategy is illustrated herein primarily with regard to a masking strategy, although it will be applicable to other polymer synthesis strategies such as the pin strategy, and the like.

In a binary synthesis strategy, the substrate is irradiated with a first mask, exposed to a first building block, irradiated with a second mask, exposed to a second building block, etc. Each combination of masked irradiation and exposure to a building block is referred to herein as a "cycle."

In a preferred binary masking strategy, the masks for each cycle allow illumination of half of a region of interest on the substrate and no illumination of the remaining half of the region of interest. By "half" it is intended herein not to mean exactly one-half the region of interest, but instead a large fraction of the region of interest such as from about 30 to 70 percent of the region of interest. It will be understood that the entire masking strategy need not take a binary form; instead non-binary cycles may be introduced as desired between binary cycles.

In the binary masking strategy, a given cycle may illuminate only about half of the region which was illuminated in a previous cycle, while not illuminating the remaining half of the illuminated portion from the previous cycle.

Conversely, a given cycle illuminates half of the region which was not illuminated in the previous cycle and does not illuminate half the region which was not illuminated in a previous cycle.

In the synthesis strategy the longest length ($\ell$) of the synthesized polymers is $\ell$ = n/a; where n is the number of cycles and a is the number of chemical building blocks (note that a given building block may be repeated).

The synthesis strategy is most readily illustrated and handled in matrix notation. At each synthesis site, the determination of whether to add a given monomer is a binary process. Therefore, each product element $P_j$ is given by the dot product of two vectors, a chemical reactant vector, e.g., C = [A,B,C,D], and a binary vector $\sigma_j$. Inspection of the products in the example below for a four-step synthesis, shows that in one four-step synthesis $\sigma_1$ = [1,0,1,0], $\sigma_2$ = [1,0,0,1], $\sigma_3$ = [0,1,1,0], and $\sigma_4$ = [0,1,0,1], where a 1 indicates illumination and a 0 indicates no illumination. Therefore, it becomes possible to build a "switch matrix" S from the column vectors $\sigma_j$ (j = l,k where k is the number of products).

| | | $\sigma_1$ | $\sigma_2$ | $\sigma_3$ | $\sigma_4$ |
|---|---|---|---|---|---|
| S | = | 1 | 1 | 0 | 0 |
| | | 0 | 0 | 1 | 1 |
| | | 1 | 0 | 1 | 0 |
| | | 0 | 1 | 0 | 1 |

The outcome P of a synthesis is simply P = CS, the product of the chemical reactant matrix and the switch matrix.

The switch matrix for an n-cycle synthesis yielding k products has n rows and k columns. An important attribute of S is that each row specifies a mask. A two-dimensional mask $m_j$ for the jth chemical step of a synthesis is obtained directly from the jth row of S by placing the elements $s_{j1}$,...$s_{jk}$ into, for example, a square format. The particular arrangement below provides a square format, although linear or other arrangements may be utilized.

$$S = \begin{matrix} s_{11} & s_{12} & s_{13} & s_{14} \\ s_{21} & s_{22} & s_{23} & s_{24} \\ s_{31} & s_{32} & s_{33} & s_{34} \\ s_{41} & s_{42} & s_{43} & s_{44} \end{matrix} \qquad m_j = \begin{matrix} s_{j1} & s_{j2} \\ s_{j3} & s_{j4} \end{matrix}$$

Of course, compounds formed in a light-activated synthesis can be positioned in any defined geometric array. A square or rectangular matrix is convenient but not required. The rows of the switch matrix may be transformed into any convenient array as long as equivalent transformations are used for each row.

For example, the masks in the four-step synthesis below are then denoted by:

$$m_1 = \begin{matrix} 1 & 1 \\ 0 & 0 \end{matrix} \qquad m_2 = \begin{matrix} 0 & 0 \\ 1 & 1 \end{matrix} \qquad m_3 = \begin{matrix} 1 & 0 \\ 1 & 0 \end{matrix} \qquad m_4 = \begin{matrix} 0 & 1 \\ 0 & 1 \end{matrix}$$

where 1 denotes illumination (activation) and 0 denotes no illumination.

The matrix representation may be used to generate a desired set of products and product maps. Each compound is defined by the product of the chemical vector and a particular switch vector. Therefore, for each synthesis address, one simply saves the switch vector, assembles all of them into a switch matrix, and extracts each of the rows to form the masks.

In some cases, particular product distributions or a maximal number of products are desired. For example, for C = [A,B,C,D], any switch vector ($\sigma_j$) consists of four bits. Sixteen four-bit vectors exist. Hence, a maximum of 16 different products can be made by sequential addition of the reagents [A,B,C,D]. These 16 column vectors can be assembled in 16! different ways to form a switch matrix. The order of the column vectors defines the masking patterns, and therefore, the spatial ordering of products but not their makeup. One ordering of these columns gives the following switch matrix (in which "null" (Ø) additions are included in brackets for the sake of completeness, although such null additions are elsewhere ignored herein):

$$
S = \begin{matrix}
\sigma 1 & & & & & & & & & & & & & & & \sigma_{16} & \\
1 & 1 & 1 & 1 & 1 & 1 & 1 & 1 & 0 & 0 & 0 & 0 & 0 & 0 & 0 & 0 & A \\
[0 & 0 & 0 & 0 & 0 & 0 & 0 & 0 & 1 & 1 & 1 & 1 & 1 & 1 & 1 & 1] & \varnothing \\
1 & 1 & 1 & 1 & 0 & 0 & 0 & 0 & 1 & 1 & 1 & 1 & 0 & 0 & 0 & 0 & B \\
[0 & 0 & 0 & 0 & 1 & 1 & 1 & 1 & 0 & 0 & 0 & 0 & 1 & 1 & 1 & 1] & \varnothing \\
1 & 1 & 0 & 0 & 1 & 1 & 0 & 0 & 1 & 1 & 0 & 0 & 1 & 1 & 0 & 0 & C \\
[0 & 0 & 1 & 1 & 0 & 0 & 1 & 1 & 0 & 0 & 1 & 1 & 0 & 0 & 1 & 1] & \varnothing \\
1 & 0 & 1 & 0 & 1 & 0 & 1 & 0 & 1 & 0 & 1 & 0 & 1 & 0 & 1 & 0 & D \\
[0 & 1 & 0 & 1 & 0 & 1 & 0 & 1 & 0 & 1 & 0 & 1 & 0 & 1 & 0 & 1] & \varnothing
\end{matrix}
$$

The columns of S according to this example are the binary representations of the numbers 15 to 0. The sixteen products of this binary synthesis are ABCD, ABC, ABD, AB, ACD, AC, AD, A, BCD, BC, BD, B, CD, C, D, and Ø (null). Also note that each of the switch vectors from the four-step synthesis masks above (and hence the synthesis products) are present in the four bit binary switch matrix. (See columns 6, 7, 10, and 11)

This synthesis procedure provides an easy way for mapping the completed products. The products in the various locations on the substrate are simply defined by the columns of the switch matrix (the first column indicating, for example, that the product ABCD will be present in the upper left-hand location of the substrate). Furthermore, if only selected desired products are to be made, the mask sequence can be derived by extracting the columns with the desired sequences. For example, to form the product set ABCD, ABD, ACD, AD, BCD, BD, CD, and D, the masks are formed by use of a switch matrix with only the 1st, 3rd, 5th, 7th, 9th, 11th, 13th, and 15th columns arranged into the switch matrix:

$$
S = \begin{matrix}
1 & 1 & 1 & 1 & 0 & 0 & 0 & 0 \\
1 & 1 & 0 & 0 & 1 & 1 & 0 & 0 \\
1 & 0 & 1 & 0 & 1 & 0 & 1 & 0 \\
1 & 1 & 1 & 1 & 1 & 1 & 1 & 1
\end{matrix}
$$

To form all of the polymers of length 4, the reactant matrix [ABCDABCDABCDABCD] is used. The switch matrix will be formed from a matrix of the binary numbers from 0 to $2^{16}$ arranged in columns. The columns having four monomers are then selected and arranged into a switch matrix. Therefore, it is seen that the binary switch matrix in general will provide a representation of all the products which can be made from an n-step synthesis, from which the desired products are then extracted.

The rows of the binary switch matrix will, in preferred embodiments, have the property that each masking step illuminates half of the synthesis area. Each masking step also factors the preceding masking step; that is, half of the region that was illuminated in the preceding step is again illuminated, whereas the other half is not. Half of the region that was not illuminated in the preceding step is also illuminated, whereas the other half is not. Thus, masking is recursive. The masks are constructed, as described previously, by extracting the elements of each row and placing them in a square array. For example, the four masks in S for a four-step synthesis are:

$$
m_1 = \begin{matrix} 1 & 1 & 1 & 1 \\ 1 & 1 & 1 & 1 \\ 0 & 0 & 0 & 0 \\ 0 & 0 & 0 & 0 \end{matrix} \quad
m_2 = \begin{matrix} 1 & 1 & 1 & 1 \\ 0 & 0 & 0 & 0 \\ 1 & 1 & 1 & 1 \\ 0 & 0 & 0 & 0 \end{matrix} \quad
m_3 = \begin{matrix} 1 & 1 & 0 & 0 \\ 1 & 1 & 0 & 0 \\ 1 & 1 & 0 & 0 \\ 1 & 1 & 0 & 0 \end{matrix} \quad
m_4 = \begin{matrix} 1 & 0 & 1 & 0 \\ 1 & 0 & 1 & 0 \\ 1 & 0 & 1 & 0 \\ 1 & 0 & 1 & 0 \end{matrix}
$$

The recursive factoring of masks allows the products of a light-directed synthesis to be represented by a polynomial. (Some light activated syntheses can only be denoted by irreducible, i.e., prime polynomials.) For example,

the polynomial corresponding to the top synthesis of Fig. 8a (discussed below) is

$$P = (A + B)(C + D)$$

A reaction polynomial may be expanded as though it were an algebraic expression, provided that the order of joining of reactants $X_1$ and $X_2$ is preserved ($X_1X_2 \neq X_2X_1$), i.e., the products are not commutative. The product then is AC + AD + BC + BD. The polynomial explicitly specifies the reactants and implicitly specifies the mask for each step. Each pair of parentheses demarcates a round of synthesis. The chemical reactants of a round (e.g., A and B) react at nonoverlapping sites and hence cannot combine with one another. The synthesis area is divided equally amongst the elements of a round (e.g., A is directed to one-half of the area and B to the other half). Hence, the masks for a round (e.g., the masks $m_A$ and $m_B$) are orthogonal and form an orthonormal set. The polynomial notation also signifies that each element in a round is to be joined to each element of the next round (e.g., A with C, A with D, B with C, and B with D). This is accomplished by having $m_C$ overlap $m_A$ and $m_B$ equally, and likewise for $m_D$. Because C and D are elements of a round, $m_C$ and $m_D$ are orthogonal to each other and form an orthonormal set.

The polynomial representation of the binary synthesis described above, in which 16 products are made from 4 reactants, is

$$P = (A + Ø)\ (B + Ø)\ (C + Ø)\ (D + Ø)$$

which gives ABCD, ABC, ABD, AB, ACD, AC, AD, A, BCD, BC, BD, B, CD, C, D, and Ø when expanded (with the rule that ØX = X and XØ = X, and remembering that joining is ordered). In a binary synthesis, each round contains one reactant and one null (denoted by Ø). Half of the synthesis area receives the reactant and the other half receives nothing. Each mask overlaps every other mask equally.

Binary rounds and non-binary rounds can be interspersed as desired, as in

$$P = (A + Ø)\ (B)\ (C + D + Ø)\ (E + F + G)$$

The 18 compounds formed are ABCE, ABCF, ABCG, ABDE, ABDF, ABDG, ABE, ABF, ABG, BCE, BCF, BCG, BDE, BDF, BDG, BE, BF, and BG. The switch matrix S for this 7-step synthesis is

$$S = \begin{matrix} 1&1&1&1&1&1&1&1&1&0&0&0&0&0&0&0&0&0 \\ 1&1&1&1&1&1&1&1&1&1&1&1&1&1&1&1&1&1 \\ 1&1&1&0&0&0&0&0&0&1&1&1&0&0&0&0&0&0 \\ 0&0&0&1&1&1&0&0&0&0&0&0&1&1&1&0&0&0 \\ 1&0&0&1&0&0&1&0&0&1&0&0&1&0&0&1&0&0 \\ 0&1&0&0&1&0&0&1&0&0&1&0&0&1&0&0&1&0 \\ 0&0&1&0&0&1&0&0&1&0&0&1&0&0&1&0&0&1 \end{matrix}$$

The round denoted by (B) places B in all products because the reaction area was uniformly activated (the mask for B consisted entirely of 1's).

The number of compounds k formed in a synthesis consisting of r rounds, in which the ith round has $b_i$ chemical reactants and $z_i$ nulls, is

$$k = \Sigma\ (b_i+z_i)$$

and the number of chemical steps n is

$$n = \Sigma b_i$$

The number of compounds synthesized when b = a (the number of chemical building blocks) and z = 0 in all rounds is $a^{n/a}$, compared with $2^n$ for a binary synthesis. For n = 20 and a = 5, 625 compounds (all tetramers) would be formed, compared with $1.049 \times 10^6$ compounds in a binary synthesis with the same number of chemical steps.

It should also be noted that rounds in a polynomial can be nested, as in

$$(A + (B+Ø) (C+Ø)) (D+Ø)$$

The products are AD, BCD, BD, CD, D, A, BC, B, C, and Ø.

Binary syntheses are attractive for two reasons. First, they generate the maximal number of products ($2^n$) for a given number of chemical steps (n). For four reactants, 16 compounds are formed in the binary synthesis, whereas only 4 are made when each round has two reactants. A 10-step binary synthesis yields 1,024 compounds, and a 20-step synthesis yields 1,048,576. Second, products formed in a binary synthesis are a complete nested set with lengths ranging from 0 to n. All compounds that can be formed by deleting one or more units from the longest product (the n-mer) are present. Contained within the binary set are the smaller sets that would be formed from the same reactants using any other set of masks (e.g., AC, AD, BC, and BD formed in the synthesis shown in Fig. 5 are present in the set of 16 formed by the binary synthesis). In some cases, however, the experimentally achievable spatial resolution may not suffice to accommodate all the compounds formed. Therefore, practical limitations may require one to select a particular subset of the possible switch vectors for a given synthesis.

1. <u>Example</u>

Fig. 5 illustrates a synthesis with a binary masking strategy. The binary masking strategy provides the greatest number of sequences for a given number of cycles. According to this example, a mask $m_1$ allows illumination of half of the substrate. The substrate is then exposed to the building block A, which binds at the illuminated regions.

Thereafter, the mask $m_2$ allows illumination of half of the previously illuminated region, while it does not illuminate half of the previously illuminated region. The building block B is then added, which binds at the illuminated regions from $m_2$.

The process continues with masks $m_3$, $m_4$, and $m_5$, resulting in the product array shown in the bottom portion of the figure. The process generates 32 (2 raised to the power of the number of monomers) sequences with 5 (the number of monomers) cycles.

2. <u>Example</u>

Fig. 6 illustrates another preferred binary masking strategy which is referred to herein as the gray code masking strategy. According to this example, the masks $m_1$ to $m_5$ are selected such that a side of any given synthesis region is defined by the edge of only one mask. The site at which the sequence BCDE is formed, for example, has its right edge defined by $m_5$ and its left side formed by mask $m_4$ (and no other mask is aligned on the sides of this site). Accordingly, problems created by misalignment, diffusion of light under the mask and the like will be minimized.

3. <u>Example</u>

Fig. 7 illustrates another binary masking strategy. According to this scheme, referred to herein as a modified gray code masking strategy, the number of masks needed is minimized. For example, the mask $m_2$ could be the same mask as m1 and simply translated laterally. Similarly, the mask $m_4$ could be the same as mask $m_3$ and simply translated laterally.

4. <u>Example</u>

A four-step synthesis is shown in Fig. 8a. The reactants are the ordered set {A,B,C,D}. In the first cycle, illumination through $m_1$ activates the upper half of the synthesis area. Building block A is then added to give the distribution 602. Illumination through mask $m_2$ (which activates the lower half), followed by addition of B yields the next intermediate distribution 604. C is added after illumination through $m_3$ (which activates the left half) giving the distribution 604, and D after illumination through $m_4$ (which activates the right half), to yield the final product pattern 608 {AC,AD, BC,BD}.

5. Example

The above masking strategy for the synthesis may be extended for all 400 dipeptides from the 20 naturally occurring amino acids as shown in Fig. 8b. The synthesis consists of two rounds, with 20 photolysis and chemical coupling cycles per round. In the first cycle of round 1, mask 1 activates 1/20th of the substrate for coupling with the first of 20 amino acids. Nineteen subsequent illumination/coupling cycles in round 1 yield a substrate consisting of 20 rectangular stripes each bearing a distinct member of the 20 amino acids. The masks of round 2 are perpendicular to round 1 masks and therefore a single illumination/coupling cycle in round 2 yields 20 dipeptides. The 20 illumination/ coupling cycles of round 2 complete the synthesis of the 400 dipeptides.

6. Example

The power of the binary masking strategy can be appreciated by the outcome of a 10-step synthesis that produced 1,024 peptides. The polynomial expression for this 10-step binary synthesis was:

$$(f+Ø)\ (Y+Ø)\ (G+Ø)\ (A+Ø)\ (G+Ø)\ (T+Ø)\ (F+Ø)\ (L+Ø)\ (S+Ø)\ (F+Ø)$$

Each peptide occupied a 400x400 μm square. A 32x32 peptide array (1,024 peptides, including the null peptide and 10 peptides of $\ell$ = 1, and a limited number of duplicates) was clearly evident in a fluorescence scan following side group deprotection and treatment with the antibody 3E7 and fluoresceinated antibody. Each synthesis site was a 400x400 μm square.

The scan showed a range of fluorescence intensities, from a background value of 3,300 counts to 22,400 counts in the brightest square (x = 20, y = 9). Only 15 compounds exhibited an intensity greater than 12,300 counts. The median value of the array was 4,800 counts.

The identity of each peptide in the array could be determined from its x and y coordinates (each range from 0 to 31) and the map of Fig. 9. The chemical units at positions 2, 5, 6, 9, and 10 are specified by the y coordinate and those at positions 1, 3, 4, 7, 8 by the x coordinate. All but one of the peptides was shorter than 10 residues. For example, the peptide at x = 12 and y = 3 is YGAGF (SEQ ID NO:3; positions 1, 6, 8, 9, and 10 are nulls). YGAFLS (SEQ ID NO: 4), the brightest element of the array, is at x = 20 and y = 9.

It is often desirable to deduce a binding affinity of a given peptide from the measured fluorescence intensity. Conceptually, the simplest case is one in which a single peptide binds to a univalent antibody molecule. The fluorescence scan is carried out after the slide is washed with buffer for a defined time. The order of fluorescence intensities is then a measure primarily of the relative dissociation rates of the antibody-peptide complexes. If the on-rate constants are the same (e.g., if they are diffusion-controlled), the order of fluorescence intensities will typically correspond to the order of binding affinities. However, the situation is sometimes more complex because a bivalent primary antibody and a bivalent secondary antibody are used. The density of peptides in a synthesis area corresponded to a mean separation of ~7 nm, which would allow multivalent antibody-peptide interactions. Hence, fluorescence intensities obtained according to the method herein will often be a qualitative indicator of binding affinity.

Another important consideration is the fidelity of synthesis. Deletions are produced by incomplete photodeprotection or incomplete coupling. The coupling yield per cycle in these experiments is typically between 85% and 95%. Implementing the switch matrix by masking is imperfect because of light diffraction, internal reflection, and scattering. Consequently, stowaways (chemical units that should not be on board) arise by unintended illumination of regions that should be dark. A binary synthesis array contains many of the controls needed to assess the fidelity of a synthesis. For example, the fluorescence signal from a synthesis area nominally containing a tetrapeptide ABCD could come from a tripeptide deletion impurity such as ACD. Such an artifact would be ruled out by the finding that the fluorescence intensity of the ACD site is less than that of the ABCD site.

The fifteen most highly fluorescent peptides in the array obtained with the synthesis of 1,024 peptides described above, were YGAFLS (SEQ ID NO:4), YGAFS (SEQ ID NO:5), YGAFL (SEQ ID NO:6), YGGFLS (SEQ ID NO: 7), YGAF (SEQ ID NO:8), YGALS (SEQ ID NO:9), YGGFS (SEQ ID NO:10), YGAL (SEQ ID NO:11), YGAFLF (SEQ ID NO:12), YGAF (SEQ ID NO:8), YGAFF (SEQ ID NO:13), YGGLS (SEQ ID NO:14), YGGFL (SEQ ID NO:1 and SEQ ID NO:15), YGAFSF (SEQ ID NO:16), and YGAFLSF (SEQ ID NO:17). A striking feature is that all fifteen begin with YG, which agrees with previous work showing that an amino-terminal tyrosine is a key determinant of binding to 3E7. Residue 3 of this set is either A or G, and residue 4 is either F or L. The exclusion of S and T from these positions is clear cut. The finding that the preferred sequence is YG (A/G) (F/L) fits nicely with the outcome of a study in which a very large library of peptides on phage generated by recombinant DNA methods was screened for binding to antibody 3E7 (see Cwirla et al., Proc. Natl. Acad. Sci. USA, (1990) 87:6378.

Additional binary syntheses based on leads from peptides on phage experiments show that YGAFMQ (SEQ ID NO:

18), YGAFM (SEQ ID NO:19), and YGAFQ (SEQ ID NO:20) give stronger fluorescence signals than does YGGFM (SEQ ID NO:21), the immunogen used to obtain antibody 3E7.

Variations on the above masking strategy will be valuable in certain circumstances. For example, if a "kernel" sequence of interest consists of PQR separated from XYZ, the aim is to synthesize peptides in which these units are separated by a variable number of different residues. The kernel can be placed in each peptide by using a mask that has 1's everywhere. The polynomial representation of a suitable synthesis is:

$$(P)\ (Q)\ (R)\ (A+\emptyset)\ (B+\emptyset)\ (C+\emptyset)\ (D+\emptyset)\ (X)\ (Y)\ (Z)$$

Sixteen peptides will be formed, ranging in length from the 6-mer PQRXYZ to the 10-mer PQRABCDXYZ.

Several other masking strategies will also find value in selected circumstances. By using a particular mask more than once, two or more reactants will appear in the same set of products. For example, suppose that the mask for an 8-step synthesis is

| | |
|---|---|
| A | 11110000 |
| B | 00001111 |
| C | 11001100 |
| D | 00110011 |
| E | 10101010 |
| F | 01010101 |
| G | 11110000 |
| H | 00001111 |

The products are ACEG, ACFG, ADEG, ADFG, BCEH, BCFH, BDEH, and BDFH. A and G always appear in the same product, although not necessarily next to each other, because their additions were directed by the same mask, and likewise for B and H.

C. Linker Selection

The linker molecules used as an intermediary between the synthesized polymers and the substrate are preferably selected for optimum length and/or type for improved binding interaction with a receptor. Diverse linkers of varying length and/or type are synthesized for subsequent attachment of a ligand. Through variations in the length and type of linker, it becomes possible to optimize the binding interaction between an immobilized ligand and its receptor.

The degree of binding between a ligand (peptide, inhibitor, hapten, drug, etc.) and its receptor (enzyme, antibody, etc.) when one of the partners is immobilized on to a substrate will in some cases depend on the accessibility of the receptor in solution to the immobilized ligand. The accessibility in turn will depend on the length and/or type of linker molecule employed to immobilize one of the partners. The VLSIPS technology is therefore preferably employed to generate an array of, preferably, inactive or inert linkers of varying length and/or type, using photochemical protecting groups to selectively expose different regions of the substrate and to build upon chemically-active groups.

In the simplest embodiment of this concept, the same unit is attached to the substrate in varying multiples or lengths in known locations on the substrate via VLSIPS techniques to generate an array of polymers of varying length. A single ligand (peptide, drug, hapten, etc.) is attached to each of them, and an assay is performed with the binding site to evaluate the degree of binding with a receptor that is known to bind to the ligand. In cases where the linker length impacts the ability of the receptor to bind to the ligand, varying levels of binding will be observed. In general, the linker which provides the highest binding will then be used to assay other ligands synthesized in accordance with the techniques herein.

The binding between a single ligand/receptor pair may be evaluated for linkers of diverse monomer sequence. linkers are synthesized in an array in accordance with the techniques herein and have different monomer sequences (and, optionally, different lengths). Thereafter, all of the linker molecules are provided with a ligand known to have at least some binding affinity for a given receptor. The given receptor is then exposed to the ligand and binding affinity is deduced. Linker molecules which provide adequate binding between the ligand and receptor are then utilized in screening studies.

D. Protecting Groups

As discussed above, selectively removable protecting groups allow creation of well defined areas of substrate

surface having differing reactivities. Preferably, the protecting groups are selectively removed from the surface by applying a specific activator, such as electromagnetic radiation of a specific wavelength and intensity. More preferably, the specific activator exposes selected areas of the surface to remove the protecting groups in the exposed areas.

Protecting groups are used in conjunction with solid phase oligomer syntheses, such as peptide syntheses using natural or unnatural amino acids, nucleotide syntheses using deoxyribonucleic and ribonucleic acids, oligosaccharide syntheses, and the like. In addition to protecting the substrate surface from unwanted reaction, the protecting groups block a reactive end of the monomer to prevent self-polymerization. For instance, attachment of a protecting group to the amino terminus of an activated amino acid, such as an N-hydroxysuccinimide-activated ester of the amino acid, prevents the amino terminus of one monomer from reacting with the activated ester portion of another during peptide synthesis. Alternatively, the protecting group may be attached to the carboxyl group of an amino acid to prevent reaction at this site. Most protecting groups can be attached to either the amino or the carboxyl group of an amino acid, and the nature of the chemical synthesis will dictate which reactive group will require a protecting group. Analogously, attachment of a protecting group to the 5'-hydroxyl group of a nucleoside during synthesis using for example, phosphate-triester coupling chemistry, prevents the 5'-hydroxyl of one nucleoside from reacting with the 3'-activated phosphate-triester of another.

Regardless of the specific use, protecting groups are employed to protect a moiety on a molecule from reacting with another reagent. Protecting groups have the following characteristics:

they prevent selected reagents from modifying the group to which they are attached; they are stable (that is, they remain attached to the molecule) to the synthesis reaction conditions; they are removable under conditions that do not adversely affect the remaining structure; and once removed, they do not react appreciably with the surface or surface-bound oligomer. The selection of a suitable protecting group will depend, of course, on the chemical nature of the monomer unit and oligomer, as well as the specific reagents they are to protect against.

Preferably, the protecting groups are photoactivatable. The properties and uses of photoreactive protecting compounds have been reviewed. See, McCray et al., Ann. Rev. of Biophys. and Biophys. Chem. (1989) 18:239-270.

Preferably, the photosensitive protecting groups will be removable by radiation in the ultraviolet (UV) or visible portion of the electromagnetic spectrum. More preferably, the protecting groups will be removable by radiation in the near UV or visible portion of the spectrum. However, activation may be performed by other methods such as localized heating, electron beam lithography, laser pumping, oxidation or reduction with microelectrodes, and the like. Sulfonyl compounds are suitable reactive groups for electron beam lithography. Oxidative or reductive removal is accomplished by exposure of the protecting group to an electric current source, preferably using microelectrodes directed to the predefined regions of the surface which are desired for activation. Other methods may also be used.

Many, although not all, of the photoremovable protecting groups will be aromatic compounds that absorb near-UV and visible radiation. Suitable photoremovable protecting groups are described in, for example, McCray et al., Patchornik, J. Amer. Chem. Soc. (1970) 92:6333, and Amit et al., J. Org. Chem. (1974) 39:192.

A preferred class of photoremovable protecting groups has the general formula:

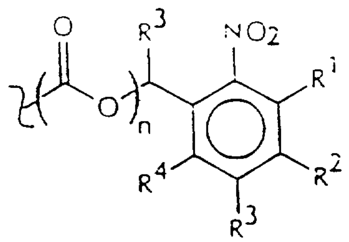

where $R^1$, $R^2$, $R^3$, and $R^4$ independently are a hydrogen atom, a lower alkyl, aryl, benzyl, halogen, hydroxyl, alkoxyl, thiol, thioether, amino, nitro, carboxyl, formate, formamido or phosphido group, or adjacent substituents (i.e., $R^1$-$R^2$, $R^2$-$R^3$, $R^3$-$R^4$) are substituted oxygen groups that together form a cyclic acetal or ketal; $R^5$ is an alkoxyl, alkyl, halo, aryl, or alkenyl group, and n = 0 or 1.

A preferred protecting group, 6-nitroveratryl (NV), which is used for protecting the carboxyl terminus of an amino acid or the hydroxyl group of a nucleotide, for example, is formed when $R^2$ and $R^3$ are each a methoxy group, $R^1$, $R^4$ and $R^5$ are each a hydrogen atom, and n = 0:

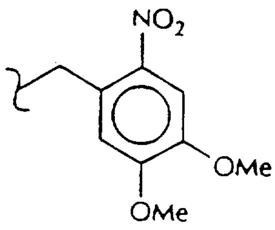

A preferred protecting group, 6-nitroveratryloxycarbonyl (NVOC), which is used to protect the amino terminus of an amino acid, for example, is formed when $R^2$ and $R^3$ are each a methoxy group, $R^1$, $R^4$ and $R^5$ are each a hydrogen atom, and n = 1:

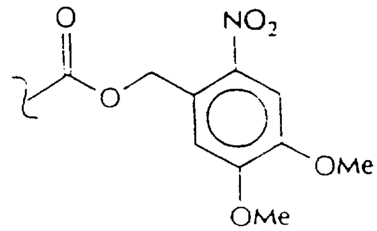

Another preferred protecting group, 6-nitropiperonyl (NP), which is used for protecting the carboxyl terminus of an amino acid or the hydroxyl group of a nucleotide, for example, is formed when $R^2$ and $R^3$ together form a methylene acetal, $R^1$, $R^4$ and $R^5$ are each a hydrogen atom, and n = 0:

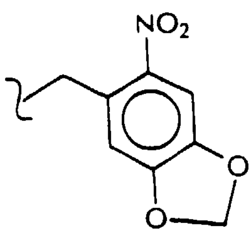

Another preferred protecting group, 6-nitropiperonyloxycarbonyl (NPOC), which is used to protect the amino terminus of an amino acid, for example, is formed when $R^2$ and $R^3$ together form a methylene acetal, $R^1$, $R^4$ and $R^5$ are each a hydrogen atom, and n = 1:

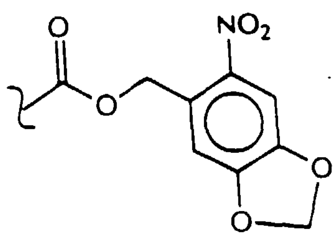

A most preferred protecting group, methyl-6-nitroveratryl (MeNV), which is used for protecting the carboxyl terminus of an amino acid or the hydroxyl group of a nucleotide, for example, is formed when $R^2$ and $R^3$ are each a methoxy group, $R^1$ and $R^4$ are each a hydrogen atom, $R^5$ is a methyl group, and n = 0:

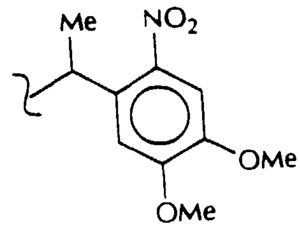

Another most preferred protecting group, methyl-6-nitroveratryloxycarbonyl (MeNVOC), which is used to protect the amino terminus of an amino acid, for example, is formed when $R^2$ and $R^3$ are each a methoxy group, $R^1$ and $R^4$ are each a hydrogen atom, $R^5$ is a methyl group, and n = 1:

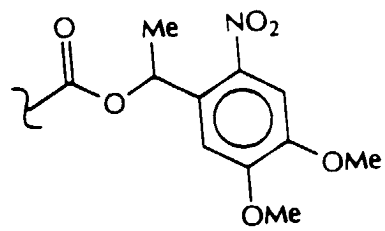

Another most preferred protecting group, methyl-6-nitropiperonyl (MeNP), which is used for protecting the carboxyl terminus of an amino acid or the hydroxyl group of a nucleotide, for example, is formed when $R^2$ and $R^3$ together form a methylene acetal, $R^1$ and $R^4$ are each a hydrogen atom, $R^5$ is a methyl group, and n = 0:

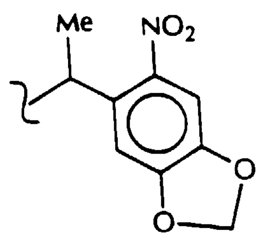

Another most preferred protecting group, methyl-6-nitropiperonyloxycarbonyl (MeNPOC), which is used to protect the amino terminus of an amino acid or to protect te 5' hydroxyl of nucleosides, for example, is formed when $R^2$ and $R^3$ together form a methylene acetal, $R^1$ and $R^4$ are each a hydrogen atom, $R^5$ is a methyl group, and n = 1:

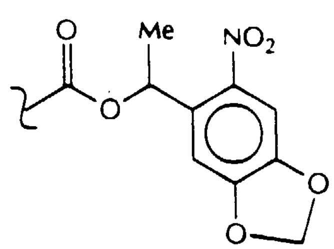

A protected amino acid having a photoactivatable oxycarbonyl protecting group, such NVOC or NPOC or their corresponding methyl derivatives, MeNVOC or MeNPOC, respectively, on the amino terminus is formed by acylating the amine of the amino acid with an activated oxycarbonyl ester of the protecting group. Examples of activated oxycarbonyl esters of NVOC and MeNVOC have the general formula:

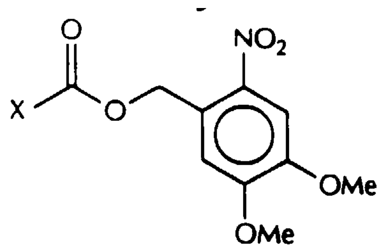

NVOC-X

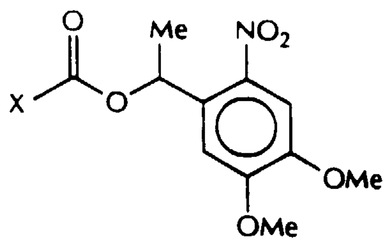

MeNVOC-X

where X is halogen, mixed anhydride, phenoxy, p-nitrophenoxy, N-hydroxysuccinimide, and the like.

A protected amino acid or nucleotide having a photoactivatable protecting group, such as NV or NP or their corresponding methyl derivatives, MeNV or MeNP, respectively, on the carboxy terminus of the amino acid or 5'-hydroxy terminus of the nucleotide, is formed by acylating the carboxy terminus or 5'-OH with an activated benzyl derivative of the protecting group. Examples of activated benzyl derivatives of MeNV and MeNP have the general formula:

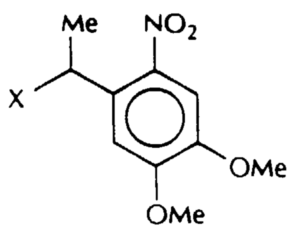

MeNV-X

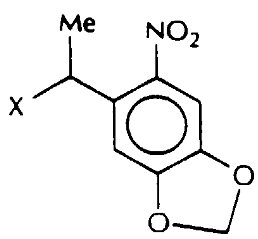

MeNP-X

where X is halogen, hydroxyl, tosyl, mesyl, trifluoromethyl, diazo, azido, and the like.

Another method for generating protected monomers is to react the benzylic alcohol derivative of the protecting group with an activated ester of the monomer. For example, to protect the carboxyl terminus of an amino acid, an activated ester of the amino acid is reacted with the alcohol derivative of the protecting group, such as 6-nitroveratrol (NVOH). Examples of activated esters suitable for such uses include halo-formate, mixed anhydride, imidazoyl formate, acyl halide, and also include formation of the activated ester in situ the use of common reagents such as DCC and the like. See Atherton et al. for other examples of activated esters.

A further method for generating protected monomers is to react the benzylic alcohol derivative of the protecting group with an activated carbon of the monomer. For example, to protect the 5'-hydroxyl group of a nucleic acid, a derivative having a 5'-activated carbon is reacted with the alcohol derivative of the protecting group, such as methyl-6-nitropiperonol (MePyROH). Examples of nucleotides having activating groups attached to the 5'-hydroxyl group have the general formula:

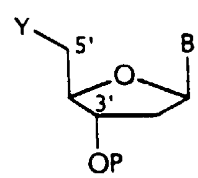

where Y is a halogen atom, a tosyl, mesyl, trifluoromethyl, azido, or diazo group, and the like.

Another class of preferred photochemical protecting groups has the formula:

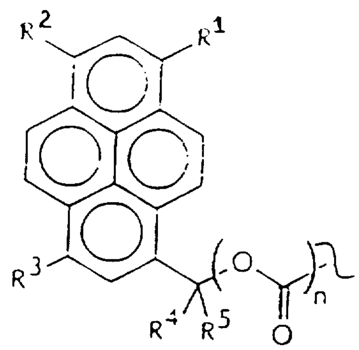

where $R^1$, $R^2$, and $R^3$ independently are a hydrogen atom, a lower alkyl, aryl, benzyl, halogen, hydroxyl, alkoxyl, thiol, thioether, amino, nitro, carboxyl, formate, formamido, sulfanates, sulfido or phosphido group, $R^4$ and $R^5$ independently are a hydrogen atom, an alkoxy, alkyl, halo, aryl, or alkenyl group, and n = 0 or 1.

A preferred protecting group, 1-pyrenylmethyloxycarbonyl (PyROC), which is used to protect the amino terminus of an amino acid, for example, is formed when $R^1$ through $R^5$ are each a hydrogen atom and n = 1:

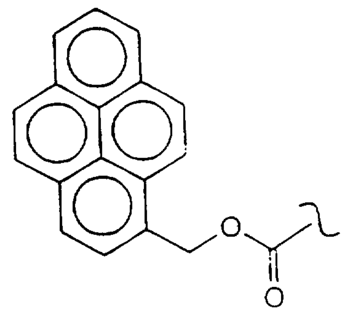

Another preferred protecting group, 1-pyrenylmethyl (PyR), which is used for protecting the carboxy terminus of an amino acid or the hydroxyl group of a nucleotide, for example, is formed when $R^1$ through $R^5$ are each a hydrogen atom and n = 0:

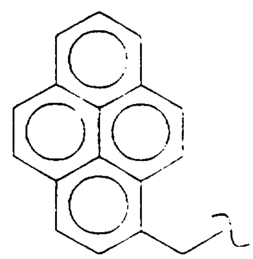

An amino acid having a pyrenylmethyloxycarbonyl protecting group on its amino terminus is formed by acylation of the free amine of amino acid with an activated oxycarbonyl ester of the pyrenyl protecting group. Examples of activated oxycarbonyl esters of PyROC have the general formula:

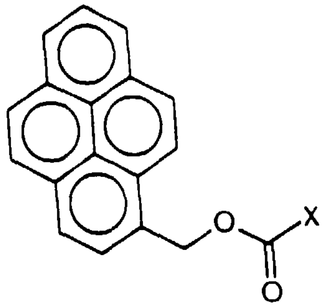

where X is halogen, or mixed anhydride, p-nitrophenoxy, or N-hydroxysuccinimide group, and the like.

A protected amino acid or nucleotide having a photoactivatable protecting group, such as PyR, on the carboxy terminus of the amino acid or 5'-hydroxy terminus of the nucleic acid, respectively, is formed by acylating the carboxy terminus or 5'-OH with an activated pyrenylmethyl derivative of the protecting group. Examples of activated pyrenylmethyl derivatives of PyROC have the general formula:

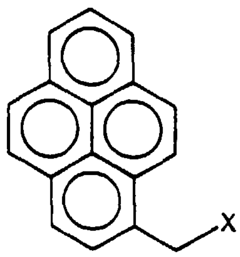

where X is a halogen atom, a hydroxyl, diazo, or azido group, and the like.

Another method of generating protected monomers is to react the pyrenylmethyl alcohol moiety of the protecting group with an activated ester of the monomer. For example, an activated ester of an amino acid can be reacted with the alcohol derivative of the protecting group, such as pyrenylmethyl alcohol (PyROH), to form the protected derivative of the carboxy terminus of the amino acid. Examples of activated esters include halo-formate, mixed anhydride, imidazoyl formate, acyl halide, and also include formation of the activated ester in situ and the use of common reagents such as DCC and the like.

Clearly, many photosensitive protecting groups are suitable for use in the present invention.

Preferably, the substrate is irradiated to remove the photoremovable protecting groups and create regions having free reactive moieties and side products resulting from the protecting group. The removal rate of the protecting groups depends on the wavelength and intensity of the incident radiation, as well as the physical and chemical properties of the protecting group itself. Preferred protecting groups are removed at a faster rate and with a lower intensity of radiation. For example, at a given set of conditions, MeNVOC and MeNPOC are photolytically removed from the N-terminus of a peptide chain faster than their unsubstituted parent compounds, NVOC and NPOC, respectively.

Removal of the protecting group is accomplished by irradiation to separate the reactive group and the degradation products derived from the protecting group. Not wishing to be bound by theory, it is believed that irradiation of an NVOC- and MeNVOC-protected oligomers occurs by the following reaction schemes:

NVOC-AA → 3,4-dimethoxy-6-nitrosobenzaldehyde + $CO_2$ + AA

MeNVOC-AA → 3,4-dimethoxy-6-nitrosoacetophenone + $CO_2$ + AA

where AA represents the N-terminus of the amino acid oligomer.

Along with the unprotected amino acid, other products are liberated into solution: carbon dioxide and a 2,3-dimethoxy-6-nitrosophenylcarbonyl compound, which can react with nucleophilic portions of the oligomer to form unwanted secondary reactions. In the case of an NVOC-protected amino acid, the degradation product is a nitrosobenzaldehyde, while the degradation product for the other is a nitrosophenyl ketone. For instance, it is believed that the product aldehyde from NVOC degradation reacts with free amines to form a Schiff base (imine) that affects the

remaining polymer synthesis. Preferred photoremovable protecting groups react slowly or reversibly with the oligomer on the support.

Again not wishing to be bound by theory, it is believed that the product ketone from irradiation of a MeNVOC-protected oligomer reacts at a slower rate with nucleophiles on the oligomer than the product aldehydes from irradiation of the same NVOC-protected oligomer. Although not unambiguously determined, it is believed that this difference in reaction rate is due to the difference in general reactivity between aldehydes and ketones towards nucleophiles due to steric and electronic effects.

The photoremovable protecting groups are readily removed. For example, the photolysis of N-protected L-phenylalanine in solution having different photoremovable protecting groups was analyzed, and the results are presented in the following table:

Table

| Photolysis of Protected L-Phe-OH | | | | |
|---|---|---|---|---|
| | $t_{1/2}$ in seconds | | | |
| Solvent | NBOC | NVOC | MeNVOC | MeNPOC |
| Dioxane | 1288 | 110 | 24 | 19 |
| 5mM $H_2SO_4$/Dioxane | 1575 | 98 | 33 | 22 |

The half life, $t_{1/2}$, is the time in seconds required to remove 50% of the starting amount of protecting group. NBOC is the 6-nitrobenzyloxycarbonyl group, NVOC is the 6-nitroveratryloxycarbonyl group, MeNVOC is the methyl-6-nitroveratryloxycarbonyl group, and MeNPOC is the methyl-6-nitropiperonyloxycarbonyl group. The photolysis was carried out in the indicated solvent with 362/364 nm-wavelength irradiation having an intensity of 10 mW/cm$^2$, and the concentration of each protected phenylalanine was 0.10 mM.

The table shows that deprotection of NVOC-, MeNVOC-, and MeNPOC-protected phenylalanine proceeded faster than the deprotection of NBOC. Furthermore, it shows that the deprotection of the two derivatives that are substituted on the benzylic carbon, MeNVOC and MeNPOC, were photolyzed at the highest rates in both dioxane and acidified dioxane.

1. Use of Photoremovable Groups During Solid-Phase Synthesis of Peptides

The formation of peptides on a solid-phase support requires the stepwise attachment of an amino acid to a substrate-bound growing chain. In order to prevent unwanted polymerization of the monomeric amino acid under the reaction conditions, protection of the amino terminus of the amino acid is required. After the monomer is coupled to the end of the peptide, the N-terminal protecting group is removed, and another amino acid is coupled to the chain. This cycle of coupling and deprotecting is continued for each amino acid in the peptide sequence. See Merrifield, J. Am. Chem. Soc. (1963) 85:2149, and Atherton et al., "Solid Phase Peptide Synthesis" 1989, IRL Press, London. As described above, the use of a photoremovable protecting group allows removal of selected portions of the substrate surface, via patterned irradiation, during the deprotection cycle of the solid phase synthesis. This selectively allows spatial control of the synthesis--the next amino acid is coupled only to the irradiated areas.

The photoremovable protecting groups may be attached to an activated ester of an amino acid at the amino terminus:

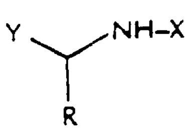

where R is the side chain of a natural or unnatural amino acid, X is a photoremovable protecting group, and Y is an activated carboxylic acid derivative. The photoremovable protecting group, X, is preferably NVOC, NPOC, PyROC, MeNVOC, MeNPOC, and the like as discussed above. The activated ester, Y, is preferably a reactive derivative having a high coupling efficiency, such as an acyl halide, mixed anhydride, N-hydroxysuccinimide ester, perfluorophenyl ester, or urethane protected acid, and the like. Other activated esters and reaction conditions are well known (See Atherton et al.).

2. Use of Photoremovable Groups During Solid-Phase Synthesis of Oligonucleotides

The formation of oligonucleotides on a solid-phase support requires the stepwise attachment of a nucleotide to a substrate-bound growing oligomer. In order to prevent unwanted polymerization of the monomeric nucleotide under the reaction conditions, protection of the 5'-hydroxyl group of the nucleotide is required. After the monomer is coupled to the end of the oligomer, the 5'-hydroxyl protecting group is removed, and another nucleotide is coupled to the chain. This cycle of coupling and deprotecting is continued for each nucleotide in the oligomer sequence. See Gait, "Oligonucleotide Synthesis: A Practical Approach" 1984, IRL Press, London.

As described above, the use of a photoremovable protecting group allows removal, via patterned irradiation, of selected portions of the substrate surface during the deprotection cycle of the solid phase synthesis. This selectively allows spatial control of the synthesis--the next nucleotide is coupled only to the irradiated areas.

Oligonucleotide synthesis generally involves coupling an activated phosphorous derivative on the 3'-hydroxyl group of a nucleotide with the 5'-hydroxyl group of an oligomer bound to a solid support. Two major chemical methods exist to perform this coupling: the phosphate-triester and phosphoramidite methods (See Gait). The described protecting groups are suitable for use in either method.

A photoremovable protecting group may be attached to an activated nucleotide on the 5'-hydroxyl group:

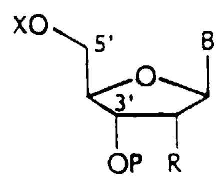

where B is the base attached to the sugar ring; R is a hydrogen atom when the sugar is deoxyribose or R is a hydroxyl group when the sugar is ribose; P represents an activated phosphorous group; and X is a photoremovable protecting group. The photoremovable protecting group, X, is preferably NV, NP, PyR, MeNV, MeNP, NVOC, NPOC, PyROC, MeNVOC, MeNPOC, and the like as described above. The activated phosphorous group, P, is preferably a reactive derivative having a high coupling efficiency, such as a phosphate-triester, phosphoramidite or the like. Other activated phosphorous derivatives, as well as reaction conditions, are well known (See Gait).

E. Amino Acid N-Carboxy Anhydrides Protected With a Photoremovable Group

During Merrifield peptide synthesis, an activated ester of one amino acid is coupled with the free amino terminus of a substrate-bound oligomer. Activated esters of amino acids suitable for the solid phase synthesis include halo-formate, mixed anhydride, imidazoyl formate, acyl halide, and also includes formation of the activated ester in situ and the use of common reagents such as DCC and the like (See Atherton et al.). A preferred protected and activated amino acid has the general formula:

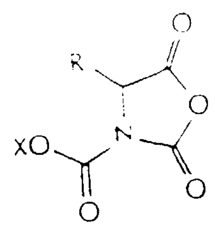

where R is the side chain of the amino acid and X is a photoremovable protecting group. This compound is a urethane-protected amino acid having a photoremovable protecting group attached to the amine. A more preferred activated amino acid is formed when the photoremovable protecting group has the general formula:

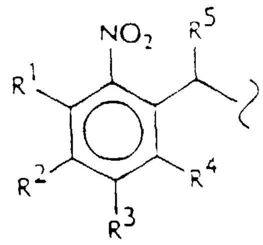

where $R^1$, $R^2$, $R^3$, and $R^4$ independently are a hydrogen atom, a lower alkyl, aryl, benzyl, halogen, hydroxyl, alkoxyl, thiol, thioether, amino, nitro, carboxyl, formate, formamido or phosphido group, or adjacent substituents (i.e., $R^1$-$R^2$, $R^2$-$R^3$, $R^3$-$R^4$) are substituted oxygen groups that together form a cyclic acetal or ketal; and $R^5$ is a hydrogen atom, alkoxyl, alkyl, halo, aryl, or alkenyl group.

A preferred activated amino acid is formed when the photoremovable protecting group is 6-nitroveratryloxycarbonyl. That is, $R^1$ and $R^4$ are each a hydrogen atom, $R^2$ and $R^3$ are each a methoxy group, and $R^5$ is a hydrogen atom. Another preferred activated amino acid is formed when the photoremovable group is 6-nitropiperonyl: $R^1$ and $R^4$ are each a hydrogen atom, $R^2$ and $R^3$ together form a methylene acetal, and $R^5$ is a hydrogen atom. Other protecting groups are possible. Another preferred activated ester is formed when the photoremovable group is methyl-6-nitroveratryl or methyl-6-nitropiperonyl.

Another preferred activated amino acid is formed when the photoremovable protecting group has the general formula:

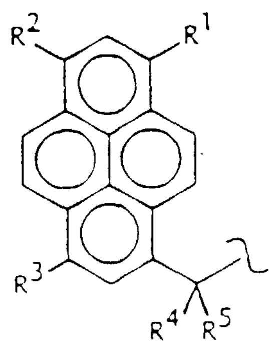

where $R^1$, $R^2$, and $R^3$ independently are a hydrogen atom, a lower alkyl, aryl, benzyl, halogen, hydroxyl, alkoxyl, thiol, thioether, amino, nitro, carboxyl, formate, formamido, sulfanate, sulfido or phosphido group, and $R^4$ and $R^5$ independently are a hydrogen atom, an alkoxy, alkyl, halo, aryl, or alkenyl group. The resulting compound is a urethane-protected amino acid having a pyrenylmethyloxycarbonyl protecting group attached to the amine. A more preferred embodiment is formed when $R^1$ through $R^5$ are each a hydrogen atom.

The urethane-protected amino acids having a photoremovable protecting group are prepared by condensation of an N-protected amino acid with an acylating agent such as an acyl halide, anhydride, chloroformate and the like (See Fuller et al., U.S. Patent No. 4,946,942 and Fuller et al., J. Amer. Chem. Soc. (1990) 112:7414-7416.

Urethane-protected amino acids having photoremovable protecting groups are generally useful as reagents during solid-phase peptide synthesis, and because of the spatial selectivity possible with the photoremovable protecting groups, are especially useful for the spatially addressing peptide synthesis. These amino acids are difunctional: the urethane group first serves to activate the carboxy terminus for reaction with the amine bound to the surface, and, once the peptide bond is formed, the photoremovable protecting group protects the newly formed amino terminus from further reaction. These amino acids are also highly reactive to nucleophiles, such as deprotected amines on the surface of the solid support, and due to this high reactivity, the solid-phase peptide coupling times are significantly reduced, and yields are typically higher.

IV. Data Collection

A. Data Collection System

Substrates prepared in accordance with the above description may be used to determine which of the plurality

of sequences thereon bind to a receptor of interest. Fig. 10 illustrates one embodiment of a device used to detect regions of a substrate which contain florescent markers. This device would be used, for example, to detect the presence or absence of a fluorescently labeled receptor such as an antibody which has bound to a synthesized polymer on a substrate.

Light is directed at the substrate from a light source 1002 such as a laser light source of the type well known to those of skill in the art such as a model no. 2025 made by Spectra Physics. Light from the source is directed at a lens 1004 which is preferably a cylindrical lens of the type well known to those of skill in the art. The resulting output from the lens 1004 is a linear beam rather than a spot of light. Thus, data can be detected substantially simultaneously along a linear array of pixels rather than on a pixel-by-pixel basis. It will be understood that while a cylindrical lens is used herein as an illustration of one technique for generating a linear beam of light on a surface, other techniques could also be utilized.

The beam from the cylindrical lens is passed through a dichroic mirror or prism and directed at the surface of the suitably prepared substrate 1008. Substrate 1008 is placed on an x-y translation stage 1009 such as a model no. PM500-8 made by Newport. Certain locations on the substrate will fluoresce and fluorescence will be transmitted along the path indicated by dashed lines back through the dichroic mirror, and focused with a suitable lens 1010 such as an f/1.4 camera lens on a linear detector 1012 via a variable f stop focusing lens 1014. Through use of a linear light beam, it becomes possible to generate data over a line of pixels (such as about 1 cm) along the substrate, rather than from individual points on the substrate. Alternatively, light is directed at a 2-dimensional area of the substrate and fluorescence is detected by a 2-dimensional CCD array. Linear detection is preferred because substantially higher power densities are obtained.

Detector 1012 detects the amount of fluorescence emitted from the substrate as a function of position. The detector may be a linear CCD array of the type commonly known to those of skill in the art. The x-y translation stage, the light source, and the detector 1012 are all operably connected to a computer 1016 such as an IBM PC-AT or equivalent for control of the device and data collection from the CCD array.

In operation, the substrate is appropriately positioned by the translation stage. The light source is then illuminated, and fluorescence intensity data are gathered with the computer via the detector.

Alternatively, the substrate and x/y translation table are placed under a microscope which includes one or more objectives. Light (about 488 nm) from a laser, which in some embodiments is a model no. 2020-05 argon ion laser manufactured by Spectraphysics, is directed at the substrate by a dichroic mirror which passes greater than about 520 nm light but reflects 488 nm light. The dichroic mirror may be, for example, a model no. FT510 manufactured by Carl Zeiss. Light reflected from the mirror then enters the microscope which may be, for example, a model no. Axioscop 20 manufactured by Carl Zeiss. Fluorescein-marked materials on the substrate will fluoresce >488 nm light, and the fluoresced light will be collected by the microscope and passed through the mirror. The fluorescent light from the substrate is then directed through a wavelength filter and, thereafter through an aperture plate. The wavelength filter may be, for example, a model no. OG530 manufactured by Melles Griot and the aperture plate may be, for example, a model no. 477352/477380 manufactured by Carl Zeiss.

The fluoresced light then enters a photomultiplier tube which in some embodiments is a model no. R943-02 manufactured by Hamamatsu, the signal is amplified in a preamplifier and photons are counted by a photon counter. The number of photons is recorded as a function of the location in the computer. The pre-amp may be, for example, a model no. SR440 manufactured by Stanford Research Systems and the photon counter may be a model no. SR400 manufactured by Stanford Research Systems. The substrate is then moved to a subsequent location and the process is repeated. In preferred embodiments the data are acquired every 1 to 100 μm with a data collection diameter of about 0.8 to 10 μm preferred. In embodiments with sufficiently high fluorescence, a CCD detector with broadfield illumination is utilized.

Fig. 11 illustrates the architecture of the data collection system in greater detail. Operation of the system occurs under the direction of the photon counting program 1102. The user inputs the scan dimensions, the number of pixels or data points in a region, and the scan speed to the counting program. Via a GPIB bus 1104 the program (in an IBM PC compatible computer, for example) interfaces with a multichannel scaler 1106 such as a Stanford Research SR 430 and an x-y stage controller 1108 such as a Newport PM500. The signal from the light from the fluorescing substrate enters a photomultiplier 1110, providing output to the scaler 1106. Data are output from the scaler indicative of the number of counts in a given region. After scanning a selected area, the stage controller is activated with commands for acceleration and velocity, which in turn drives the scan stage 1112 such as a Newport PM500-A to another region.

Data are collected in an image data file 1114 and processed in a scaling program 1116. A scaled image is output for display on, for example, a VGA display 1118. The image is scaled based on an input of the percentage of pixels to clip and the minimum and maximum pixel levels to be viewed. The system outputs for use the min and max pixel levels in the raw data.

B. Data Analysis

The output from the data collection system is an array of data indicative of fluorescence intensity versus location on the substrate. The data are typically taken over regions substantially smaller than the area in which synthesis of a given polymer has taken place. Merely by way of example, if polymers were synthesized in squares on the substrate having dimensions of 500 microns by 500 microns, the data may be taken over regions having dimensions of 5 microns by 5 microns. Preferably the regions over which florescence data are taken across the substrate are less than about 1/2 the area of the regions in which individual polymers are synthesized, preferably less than 1/10 the area in which a single polymer is synthesized, and most preferably less than 1/100 the area in which a single polymer is synthesized. Hence, within any area in which a given polymer has been synthesized, a large number of fluorescence data points are collected.

A plot of the number of pixels versus fluorescence intensity for a scan of a cell when it has been exposed to, for example, a labeled antibody will typically take the form of a bell curve, but spurious data are observed, particularly at higher intensities. Since it is desirable to use an average of fluorescence intensity over a given synthesis region in determining relative binding affinity, these spurious data will tend to undesirably skew the data.

Accordingly, the data may be corrected for removal of these spurious data points, and an average of the data points is thereafter utilized in determining relative binding efficiency.

Fig. 12 illustrates one example of a system for removal of spurious data from a set of fluorescence data such as data used in affinity screening studies. A user or the system inputs data relating to the chip location and cell corners at step 1302. From this information and the image file, the system creates a computer representation of a histogram at step 1304, the histogram (at least in the form of a computer file) plotting number of data pixels versus intensity.

For each cell, a main data analysis loop is then performed. For each cell, at step 1306, the system calculates the total fluorescence intensity or number of pixels for the bandwidth centered around varying intensity levels. For example, as shown in the plot to the right of step 1306, the system calculates the number of pixels within the band of width w. The system then "moves" this bandwidth to a higher center intensity, and again calculates the number of pixels in the bandwidth. This process is repeated until the entire range of intensities have been scanned, and at step 1308 the system determines which band has the highest total number of pixels. The data within this bandwidth are used for further analysis. Assuming the bandwidth is selected to be reasonably small, this procedure will have the effect of eliminating spurious data located at the higher intensity levels. The system then repeats at step 1310 if all cells have been evaluated, or repeats for the next cell.

At step 1312 the system then integrates the data within the bandwidth for each of the selected cells, sorts the data at step 1314 using the synthesis procedure file, and displays the data to a user on, for example, a video display or a printer.

V. Representative Applications

A. Oligonucleotide Synthesis

The generality of light directed spatially addressable parallel chemical synthesis is demonstrated by application to nucleic acid synthesis.

1. Example

Light activated formation of a thymidine-cytidine dimer was carried out. A three dimensional representation of a fluorescence scan showing a 7 square by 4 square checkerboard pattern generated by the light-directed synthesis of a dinucleotide was produced. 5'-nitroveratryl thymidine was attached to a synthesis substrate through the 3' hydroxyl group. The nitroveratryl protecting groups were removed by illumination through a 500 μm checkerboard mask. The substrate was then treated with phosphoramidite activated 2'-deoxycytidine. In order to follow the reaction fluorometrically, the deoxycytidine had been modified with an FMOC protected aminohexyl linker attached to the exocyclic amine (5'-O-dimethoxytrityl-4-N-(6-N-fluorenylmethylcarbamoyl-hexylcarboxy)-2'-deoxycytidine). After removal of the FMOC protecting group with base, the regions which contained the dinucleotide were fluorescently labelled by treatment of the substrate with 1 mM FITC in DMF for one hour.

The three-dimensional representation of the fluorescence intensity data showing alternating squares of bright raised pixels reproduces the checkerboard illumination pattern used during photolysis of the substrate. This result demonstrates that oligonucleotides as well as peptides can be synthesized by the light-directed method.

In another example the light-activated formation of thymidine-cytidine-cytidine was carried out as shown in Fig. 13. Here, as in the previous example, 5'-nitroveratryl thymidine was attached to the substrate, via phosphoramidite chemistry to a surface containing [Bis (2-hydroxyethyl)-3-aminopropylsiloxane]. The slide was then uniformly illumi-

nated (362nm at ~ 14mW/cm$^2$) for 10 minutes in the presence of dioxane. After drying, the surface was then treated with N,4-dimethoxytrityl-5'-nitroveratryl-2'-deoxycytidine-3'-O-(2-cyanoethyl)-N,N-diisopropylphosphoramidite in the presence of tetrazole (standard phosphoramidite coupling chemistry). After oxidizing and drying, the plate was again illuminated as before except that a 500 µm checkerboard mask was placed between the light source and the slide. The surface was then exposed to 5'-O-(4,4'-Dimethoxy)-N-4-(6-((Biotinoyl)amino)hexanoyl)amino)hexanoyl, aminohexyl)-5-methyl-2'-deoxycytidine-3'-O-(2-cyanoethyl)-N,N-diisopropylphosphoramidite with tetrazale. After oxidizing and drying, the areas which contained the trinucleotide were fluroescently labelled by treatment with FITC labled streptavidin. A resulting representation of the fluorescence intensity data showed alternating bright and dark squares corresponding to the 500 µm and checkerboard illumination pattern used during photolysis.

VI. Conclusion

The inventions herein provide a new approach for the simultaneous synthesis of a large number of compounds. The method can be applied whenever one has chemical building blocks that can be coupled in a solid-phase format, and when light can be used to generate a reactive group.

The above description is illustrative and not restrictive. Many variations of the invention will become apparent to those of skill in the art upon review of this disclosure. Merely by way of example, while the invention is illustrated primarily with regard to peptide and nucleotide synthesis, the invention is not so limited. The scope of the invention should, therefore, be determined not with reference to the above description, but instead should be determined with reference to the appended claims.

**Claims**

**1.** A compound having the formula:

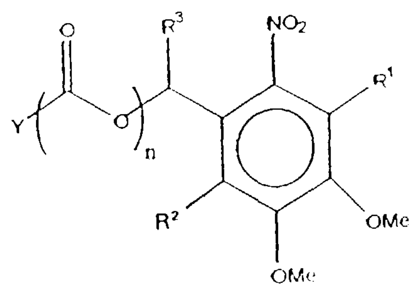

wherein n = 0 or 1; Y is a functional group; $R^1$ and $R^2$ independently are a hydrogen atom, an aryl, benzyl, halogen, hydroxyl, alkoxyl, thiol, thioether, amino, nitro, carboxyl, formate, formamido, sulfido, or phosphido group; and $R^3$ is a alkoxy, alkyl, aryl, or alkenyl group.

**2.** A compound having the formula:

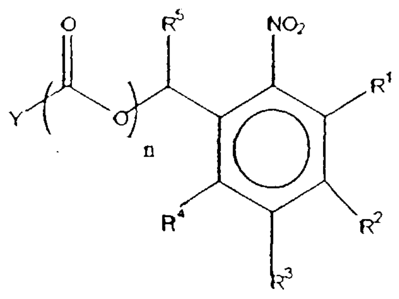

wherein n = 0 or 1; Y is a C-5' oxygen group of a natural or unnatural deoxyribonucleic and ribonucleic acid; $R^1$ through $R^4$ independently are a hydrogen atom, a methyl ($R^3$ only), an aryl, benzyl, halogen, hydroxyl, alkoxyl, thiol, thioether, amino, nitro, carboxyl, formate, formamido, sulfido, or phosphido group; and $R^5$ is an alkoxy, alkyl, aryl, or alkenyl group; provided that (i) $R^2$ and $R^3$ are not both methoxy when $R^1$ and $R^4$ are hydrogen and $R^5$ is 2-nitrophenyl or 3,4-dimethoxy-6 nitrophenyl, and (ii) $R^1$, $R^2$, $R^3$ and $R^4$ are not hydrogen simultaneously.

**3.** A compound of claim 2, wherein $R^1$ and $R^4$ are selected independently from methoxy, hydrogen, alkoxy, and halogen.

**4.** A compound having the formula:

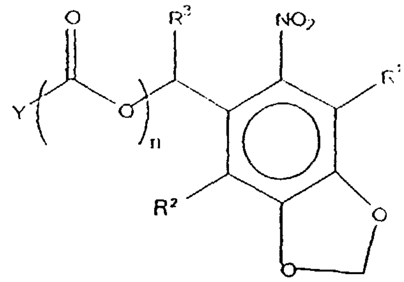

wherein n = 0 or 1; Y is a functional group; $R^1$ and $R^2$ independently are a hydrogen atom, an aryl, benzyl, halogen, hydroxyl, alkoxyl, thiol, thioether, amino, nitro, carboxyl, formate, formamido, sulfido, or phosphido group; and $R^3$ is alkoxy, alkyl, aryl, or alkenyl.

**5.** A compound of claim 1 or claim 4 wherein $R^1$ and $R^2$ are chosen independently from methoxy, hydrogen, alkoxy, and halogen.

**6.** A compound of any one of claims 1 to 4, wherein in the case of a compound of claim 2 or claim 3 $R^5$ is methyl and for any other compound $R^3$ is methyl.

**7.** A compound of any one of claims 1 to 6, wherein Y is a functional group of a molecule selected from natural and unnatural amino acids and peptides, nucleosides, and oligonucleotides.

**8.** The use of a compound of any one of claims 1, 4 or 5 or of claim 6 or claim 7 when directly or indirectly dependent upon any one of claims 1, 4 or 5 to provide protecting groups in an ordered method for forming a plurality of polymer sequences by sequential addition of reagents comprising the step of serially protecting and deprotecting portions of said plurality of polymer sequences for addition of other portions of said polymer sequences using a binary synthesis strategy.

**9.** The use of claim 8 wherein either:

(a) said binary synthesis strategy is a binary masking strategy, and wherein said masking strategy provides at least two consecutive steps in which a mask factors a previous mask by protecting a portion of a previously illuminated portion from light and exposing a portion of a previously protected portion to light; or

(b) said binary synthesis strategy is a binary masking strategy and wherein said masks are arranged in a gray code masking strategy, said gray code masking strategy having one edge illumination on each of a plurality of synthesis sites.

**10.** The use of claim 9 further comprising the step of forming a portion of said polymers with a non-binary masking strategy.

**Patentansprüche**

**1.** Eine Verbindung der allgemeinen Formel

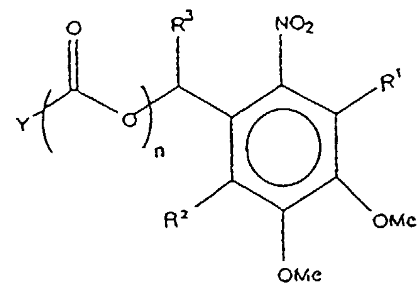

worin n = 0 oder 1 ist, Y eine funktionelle Gruppe ist, $R^1$ und $R^2$ unabhängig ein Wasserstoffatom, eine Aryl-, Benzyl-, Halogen-, Hydroxyl-, Alkoxyl-, Thiol-, Thioether-, Amino-, Nitro-, Carboxyl-, Formiat-, Formamido-, Sulfido- oder Phosphidogruppe sind und $R^3$ eine Alkoxy-, Alkyl-, Aryl- oder Alkenylgruppe ist.

**2.** Eine Verbindung der allgemeinen Formel

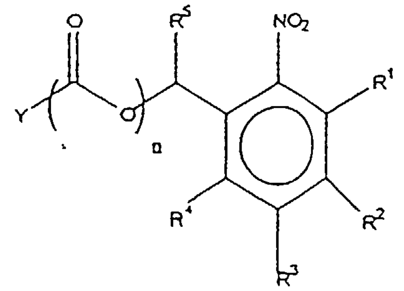

worin n = 0 oder 1 ist, Y eine C-5'-Sauerstoffgruppe einer natürlichen oder nicht-natürlichen Desoxyribonuclein- und Ribonucleinsäure ist, $R^1$ bis $R^4$ unabhängig ein Wasserstoffatom, eine Methyl- (nur $R^3$), Aryl-, Benzyl-, Halogen-, Hydroxyl-, Alkoxyl-, Thiol-, Thioether-, Amino- Nitro-, Carboxyl-, Formiat-, Formamido-, Sulfido- oder Phosphidogruppen sind und $R^5$ eine Alkoxy-, Alkyl-, Aryl- oder Alkenylgruppe ist, mit der Maßgabe, dass (i) $R^2$ und $R^3$ nicht beide Methoxygruppen sind, wenn $R^1$ und $R^4$ Wasserstoffatome sind und $R^5$ eine 2-Nitrophenyl- oder 3,4-Dimethoxy-6-nitrophenylgruppe ist und (ii) $R^1$, $R^2$, $R^3$ und $R^4$ nicht gleichzeitig Wasserstoffatome sind.

**3.** Verbindung nach Anspruch 2, wobei $R^1$ und $R^4$ unabhängig aus einer Methoxygruppe, einem Wasserstoffatom, einer Alkoxy- und Halogengruppe ausgewählt sind.

**4.** Eine Verbindung der allgemeinen Formel

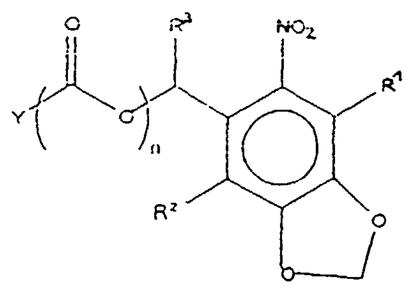

worin n = 0 oder 1 ist, Y eine funktionelle Gruppe ist, $R^1$ und $R^2$ unabhängig ein Wasserstoffatom, eine Aryl-, Benzyl-, Halogen-, Hydroxyl-, Alkoxyl-, Thiol-, Thioether-, Amino-, Nitro-, Carboxyl-, Formiat-, Formamido-, Sulfido- oder Phosphidogruppe sind und $R^3$ eine Alkoxy-, Alkyl-, Aryl- oder Alkenylgruppe ist.

**5.** Verbindung nach Anspruch 1 oder 4, wobei $R^1$ und $R^2$ unabhängig aus einer Methoxygruppe, einem Wasserstoffatom, einer Alkoxy- und Halogengruppe ausgewählt sind.

6. Verbindung nach einem der Ansprüche 1 bis 4, wobei im Fall einer Verbindung nach Anspruch 2 oder 3 $R^5$ eine Methylgruppe und für eine beliebige andere Verbindung $R^3$ eine Methylgruppe ist.

7. Verbindung nach einem der Ansprüche 1 bis 6, wobei Y eine funktionelle Gruppe eines Moleküls ist, das aus natürlichen und nicht-natürlichen Aminosäuren und Peptiden, Nucleosiden und Oligonucleotiden ausgewählt ist.

8. Verwendung einer Verbindung nach einem der Ansprüche 1, 4 oder 5 oder nach Anspruch 6 oder 7, wenn diese direkt oder indirekt von einem der Ansprüche 1, 4 oder 5 abhängen, um Schutzgruppen in einem geordneten Verfahren zur Herstellung einer Vielzahl von Polymersequenzen durch aufeinanderfolgendes Zugeben von Reagentien bereitzustellen, umfassend den Schritt des aufeinanderfolgenden Schützens und Entschützens von Abschnitten der Vielzahl von Polymersequenzen zur Addition anderer Abschnitte der Polymersequenzen unter Verwendung einer binären Synthesestrategie.

9. Verwendung nach Anspruch 8, wobei entweder

(a) die binäre Synthesestrategie eine binäre Maskierungsstrategie ist und wobei die Maskierungsstrategie mindestens zwei aufeinanderfolgende Schritte bereitstellt, bei denen eine Maske eine vorhergehende Maske faktorisiert, und zwar durch Schützen eines Abschnitts eines vorher belichteten Abschnitts vor Licht und Belichten eines Abschnitts eines vorher geschützten Abschnitts, oder

(b) die binäre Synthesestrategie eine binäre Maskierungsstrategie ist und wobei die Masken in einer Graucode-Maskierungsstrategie angeordnet sind, wobei die Graucode-Maskierungsstrategie eine Kantenbelichtung auf jeder einer Vielzahl von Synthesestellen aufweist.

10. Verwendung nach Anspruch 9, weiter umfassend den Schritt des Herstellens eines Abschnitts der Polymere mittels einer nicht-binären Maskierungstrategie.

**Revendications**

1. Composé de formule :

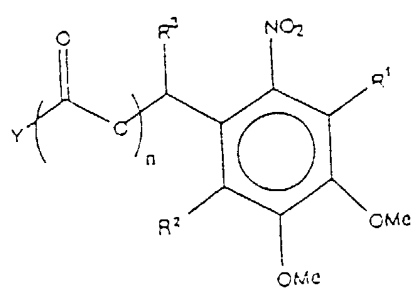

dans laquelle n = 0 ou 1 ; Y est un groupe fonctionnel ; $R^1$ et $R^2$ représentent indépendamment un atome d'hydrogène, un atome d'halogène, un groupe aryle, benzyle, hydroxy, alkoxy, thiol, thioéther, amino, nitro, carboxyle, formate, formamido, sulfido ou phosphido ; et $R^3$ est un groupe alkoxy, alkyle, aryle ou alcényle.

2. Composé de formule :

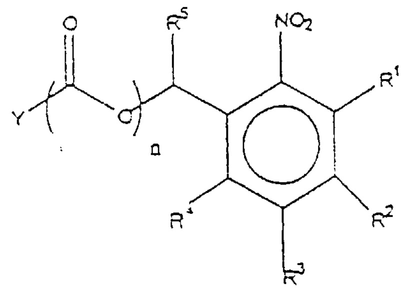

dans laquelle n = 0 ou 1; Y est un groupe dérivé d'un acide désoxyribonucléique ou ribonucléique naturel ou synthétique rattaché par l'atome d'oxygène en C-5' ; $R^1$ à $R^4$ représentent indépendamment un atome d'hydrogène, un atome d'halogène, un groupe méthyle ($R^3$ seulement), aryle, benzyle, hydroxy, alcoxy, thiol, thioéther, amino, nitro, carboxyle, formate, formamido, sulfido ou phosphido ; et $R^5$ est un groupe alcoxy, alkyle, aryle ou alcényle ; étant entendu que (i) $R^2$ et $R^3$ ne sont pas tous les deux méthoxy lorsque $R^1$ et $R^4$ sont des atomes d'hydrogène et $R^5$ est un groupe 2-nitrophényle ou 3,4-diméthoxy-6-nitrophényle, et (ii) $R^1$, $R^2$, $R^3$ et $R^4$ ne sont pas simultanément des atomes d'hydrogène.

**3.** Composé selon la revendication 2, dans lequel $R^1$ à $R^4$ sont indépendamment choisis parmi méthoxy, un atome d'hydrogène, alcoxy et un atome d'halogène.

**4.** Composé de formule :

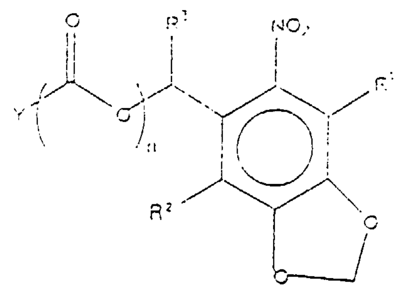

dans laquelle n = 0 ou 1 ; Y est un groupe fonctionnel ; $R^1$ et $R^2$ sont indépendamment un atome d'hydrogène, un atome d'halogène, un groupe aryle, benzyle, hydroxy, alkoxy, thiol, thioéther, amino, nitro, carboxyle, formate, formamido, sulfido ou phosphido ; et $R^3$ est un groupe alkoxy, alkyle, aryle ou alcényle.

**5.** Composé selon la revendication 1 ou la revendication 4, dans lequel $R^1$ et $R^2$ représentent indépendamment un groupe méthoxy, un atome d'hydrogène, un groupe alcoxy ou un atome d'halogène.

**6.** Composé selon l'une quelconque des revendications 1 à 4, dans lequel, dans le cas d'un composé selon la revendication 2 ou la revendication 3, $R^5$ représente méthyle, et dans tous les autres cas $R^3$ représente méthyle.

**7.** Composé selon l'une quelconque des revendications 1 à 6, dans lequel Y représente un groupe fonctionnel ou une molécule choisie parmi les peptides et aminoacides, les nucléosides et les oligonucléotides, naturels et synthétiques.

**8.** Utilisation d'un composé selon l'une quelconque des revendications 1, 4 ou 5 ou selon la revendication 6 ou selon la revendication 7 lorsqu'elles sont directement ou indirectement dépendantes de l'une des revendications 1, 4 ou 5, en vue de fournir des groupes protecteurs dans une méthode ordonnée visant à former une pluralité de séquences polymères par addition séquentielle de réactifs comprenant l'étape consistant à protéger en série et à déprotéger des parties de ladite pluralité de séquences polymères en vue de l'addition d'autres parties desdites séquences de polymères par utilisation d'une stratégie de synthèse binaire.

**9.** Utilisation selon la revendication 8, selon laquelle soit :

(a) ladite stratégie de synthèse binaire est une stratégie de masquage binaire, et, selon laquelle ladite stratégie de masquage fournit au moins deux étapes consécutives dans lesquelles un masque factorise un masque précédent en protégeant de la lumière une partie d'une partie précédemment irradiée et en exposant à la lumière une partie d'une partie précédemment protégée ; soit
(b) ladite stratégie de synthèse binaire est une stratégie de masquage binaire, et, selon laquelle lesdits masques sont disposés suivant une stratégie de masquage à échelons, ladite stratégie de masquage à échelons présentant une irradiation en coin sur chacun des multiples sites de synthèse.

**10.** Utilisation selon la revendication 9 comprenant en outre l'étape consistant à former une partie desdits polymères par une stratégie de masquage non binaire.

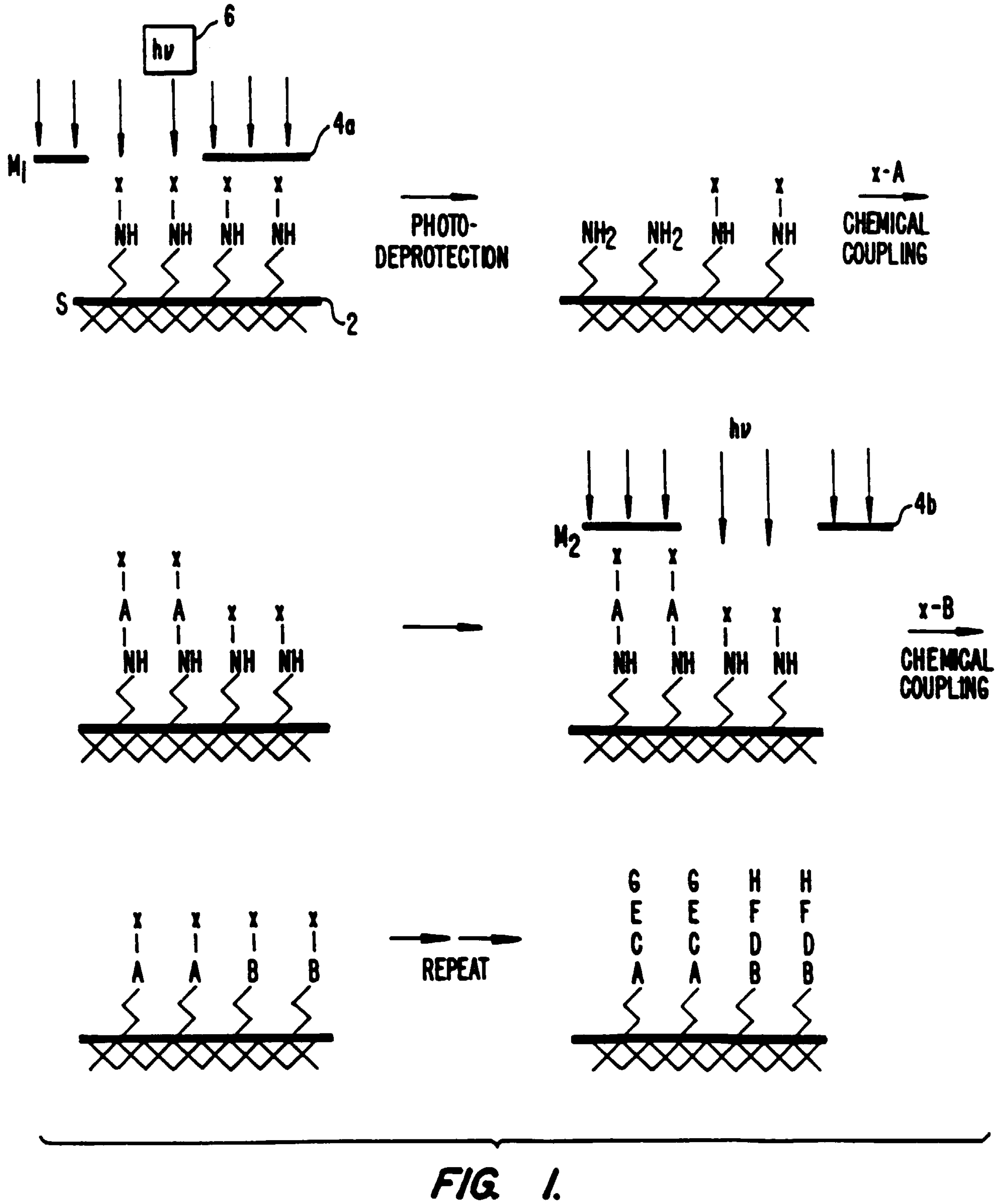
6
hν
4a
M1
x
NH
S
2
PHOTO-
DEPROTECTION
NH2
x-A
CHEMICAL
COUPLING
A
hν
4b
M2
x-B
CHEMICAL
COUPLING
B
REPEAT
G
E
C
H
F
D

FIG. 1.

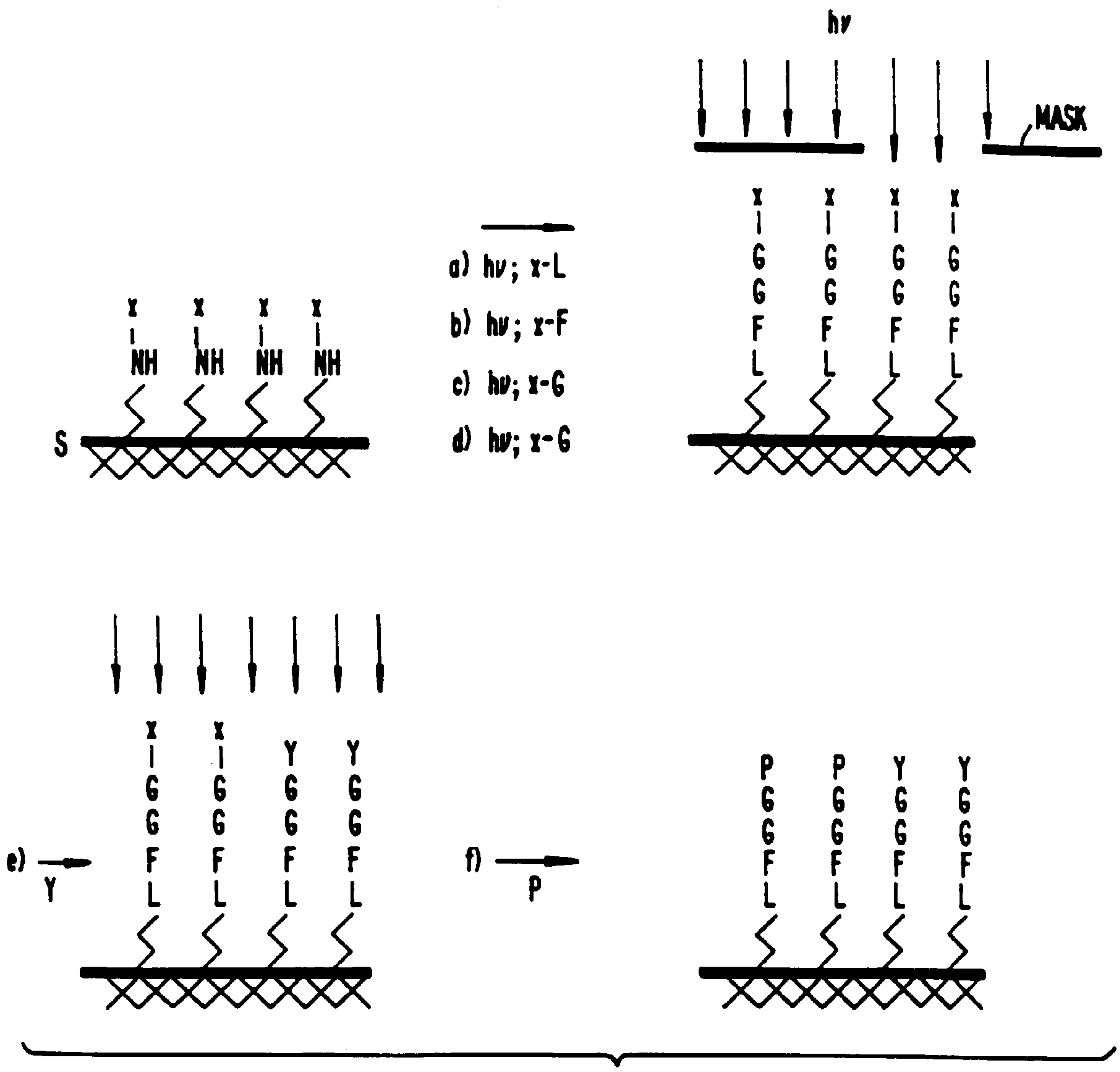


FIG. 2.

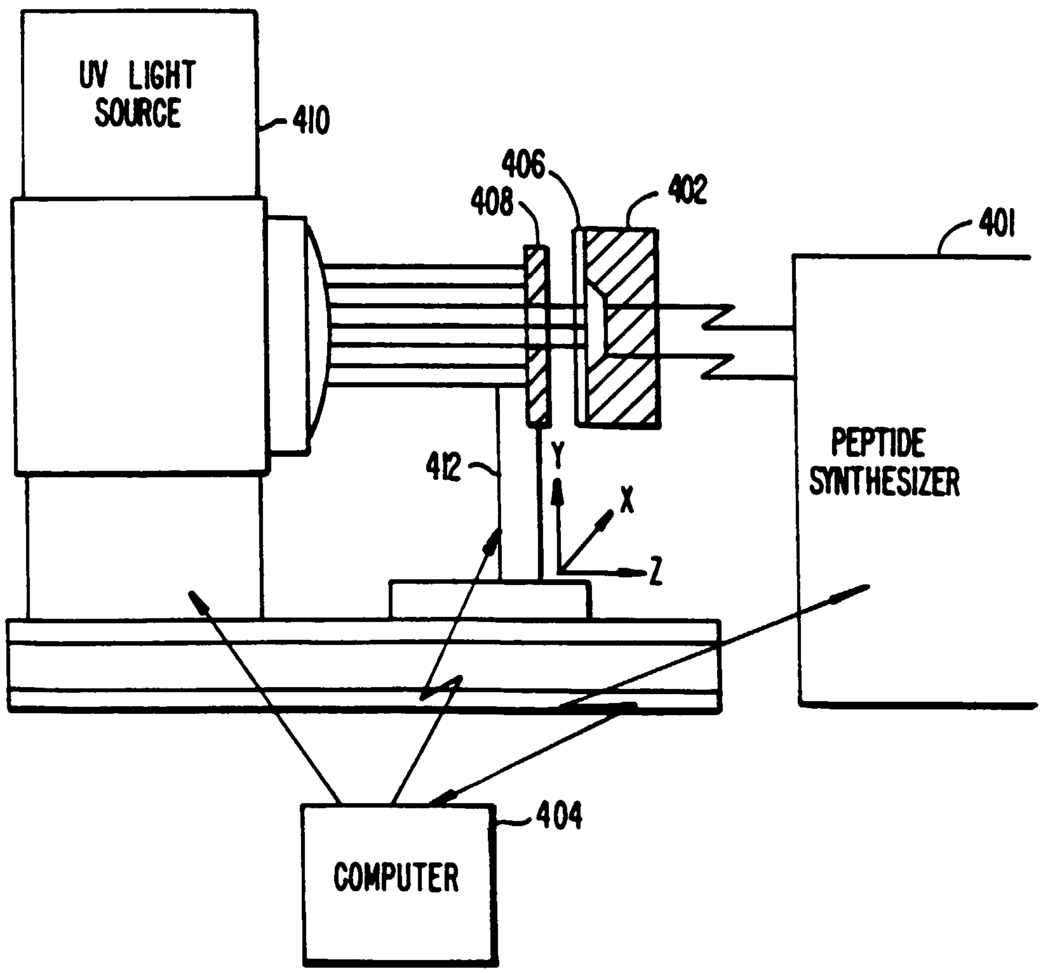
UV LIGHT SOURCE
410
406
402
408
401
PEPTIDE SYNTHESIZER
412
Y
X
Z
404
COMPUTER

FIG. 3.

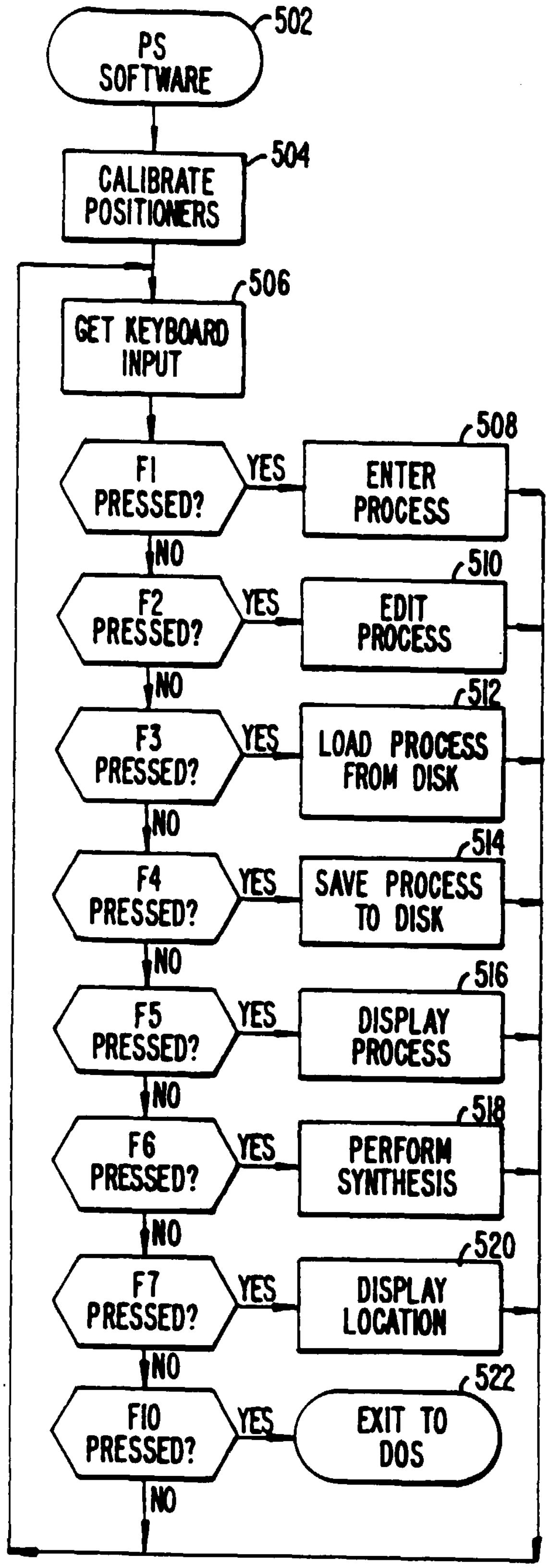
PS SOFTWARE
502
CALIBRATE POSITIONERS
504
GET KEYBOARD INPUT
506
F1 PRESSED?
YES
ENTER PROCESS
508
NO
F2 PRESSED?
YES
EDIT PROCESS
510
NO
F3 PRESSED?
YES
LOAD PROCESS FROM DISK
512
NO
F4 PRESSED?
YES
SAVE PROCESS TO DISK
514
NO
F5 PRESSED?
YES
DISPLAY PROCESS
516
NO
F6 PRESSED?
YES
PERFORM SYNTHESIS
518
NO
F7 PRESSED?
YES
DISPLAY LOCATION
520
NO
F10 PRESSED?
YES
EXIT TO DOS
522
NO

FIG. 4A.

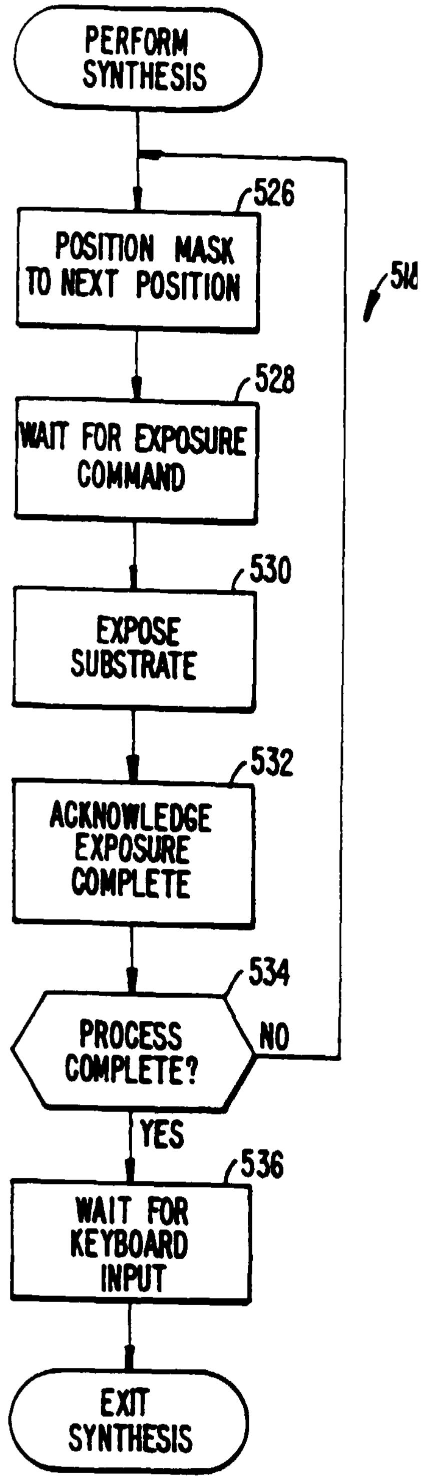
PERFORM SYNTHESIS
POSITION MASK TO NEXT POSITION
526
WAIT FOR EXPOSURE COMMAND
528
EXPOSE SUBSTRATE
530
ACKNOWLEDGE EXPOSURE COMPLETE
532
PROCESS COMPLETE?
534
NO
YES
WAIT FOR KEYBOARD INPUT
536
EXIT SYNTHESIS

FIG. 4B.

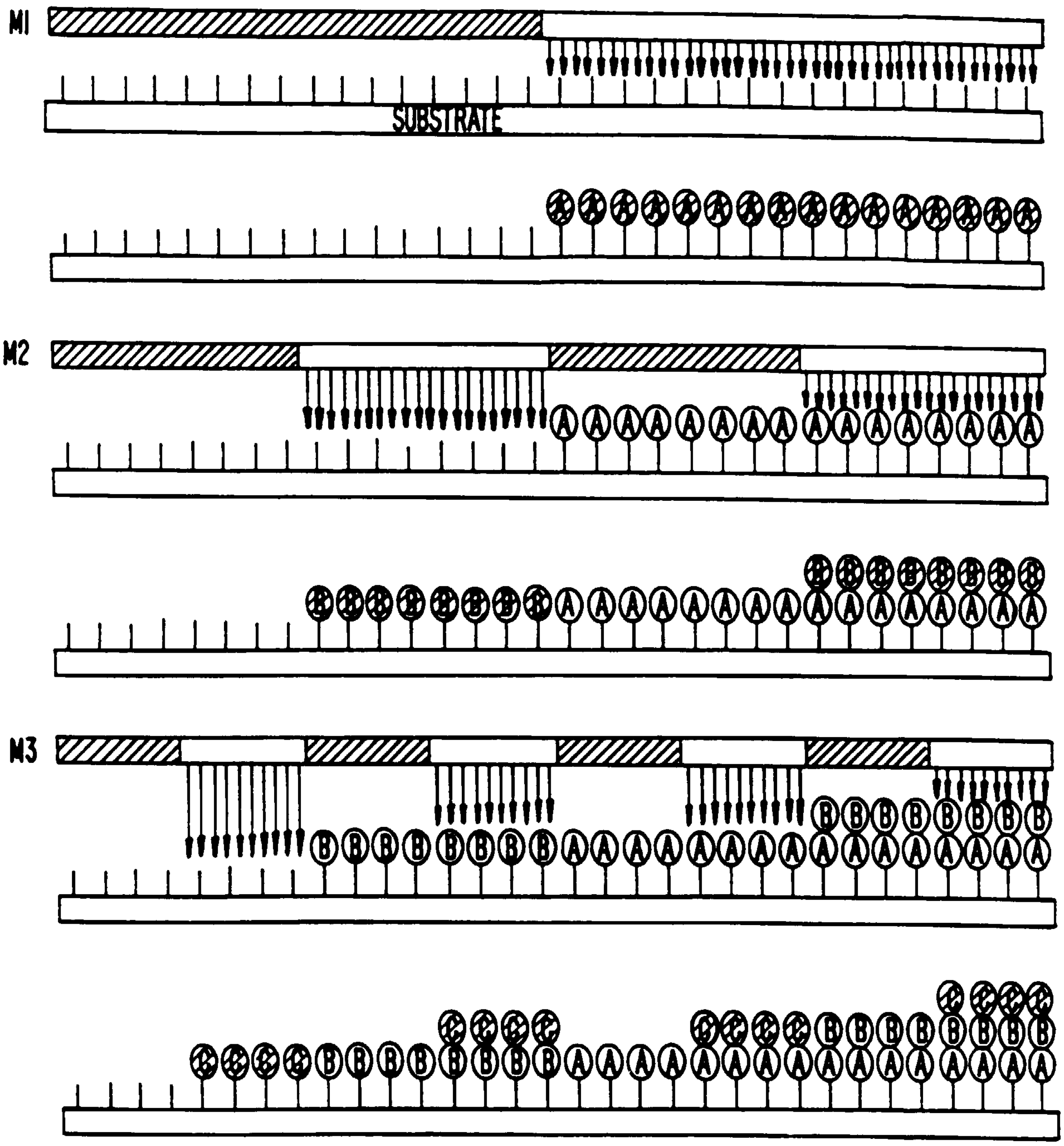


FIG. 5A.

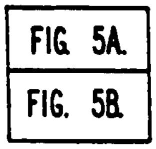


FIG. 5.

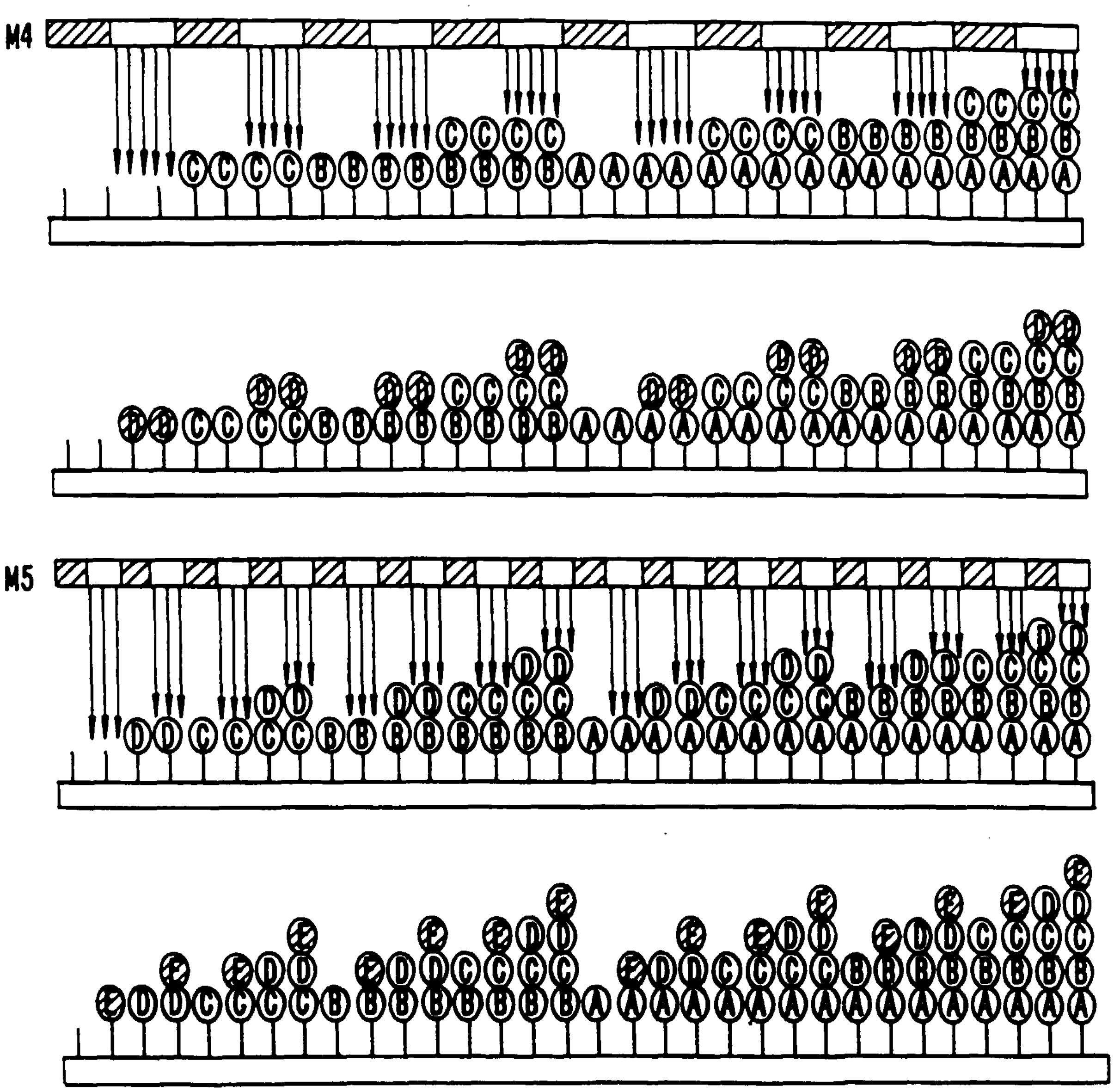


FIG. 5B.

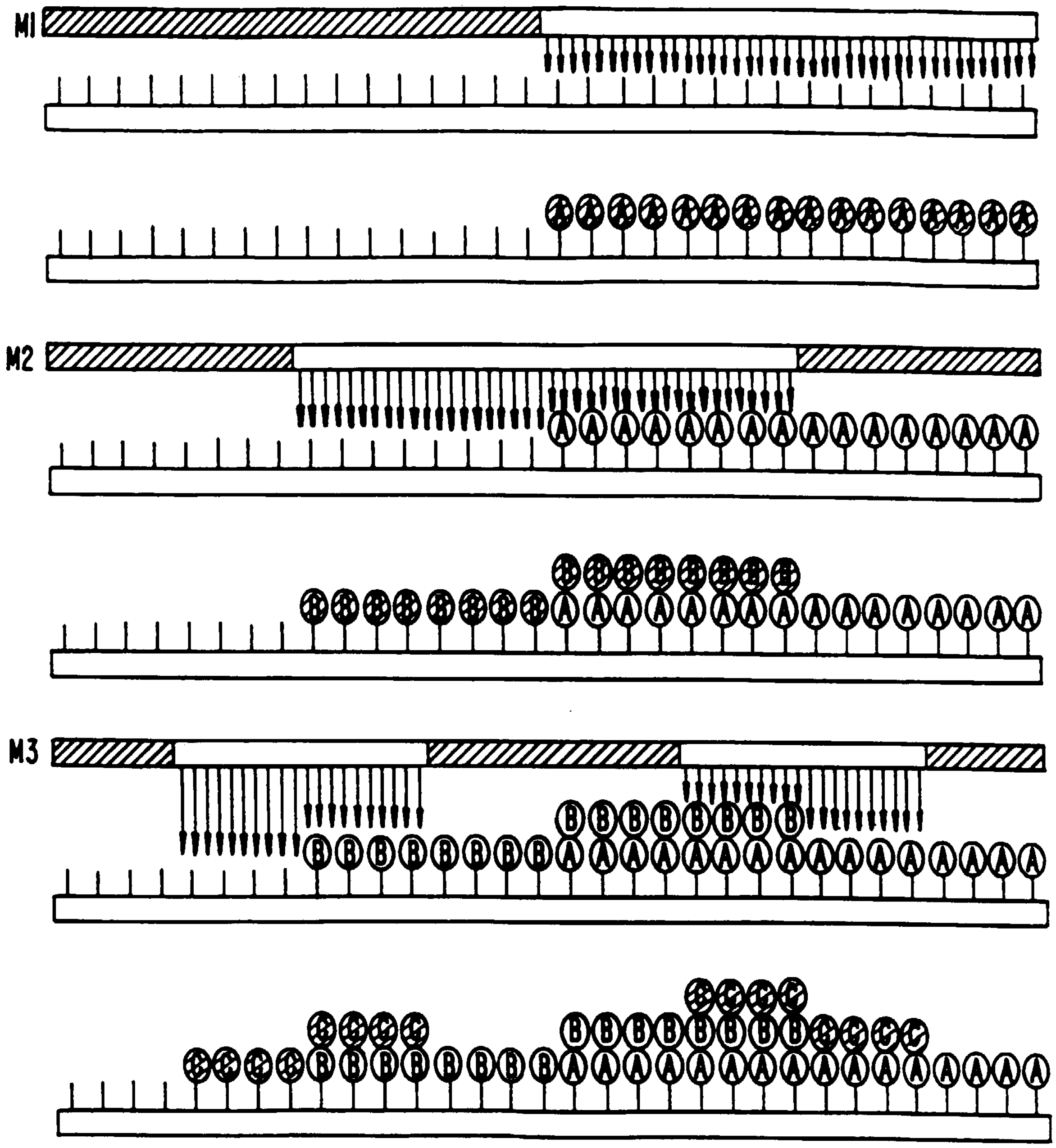


FIG. 6A.

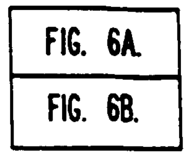


FIG. 6.

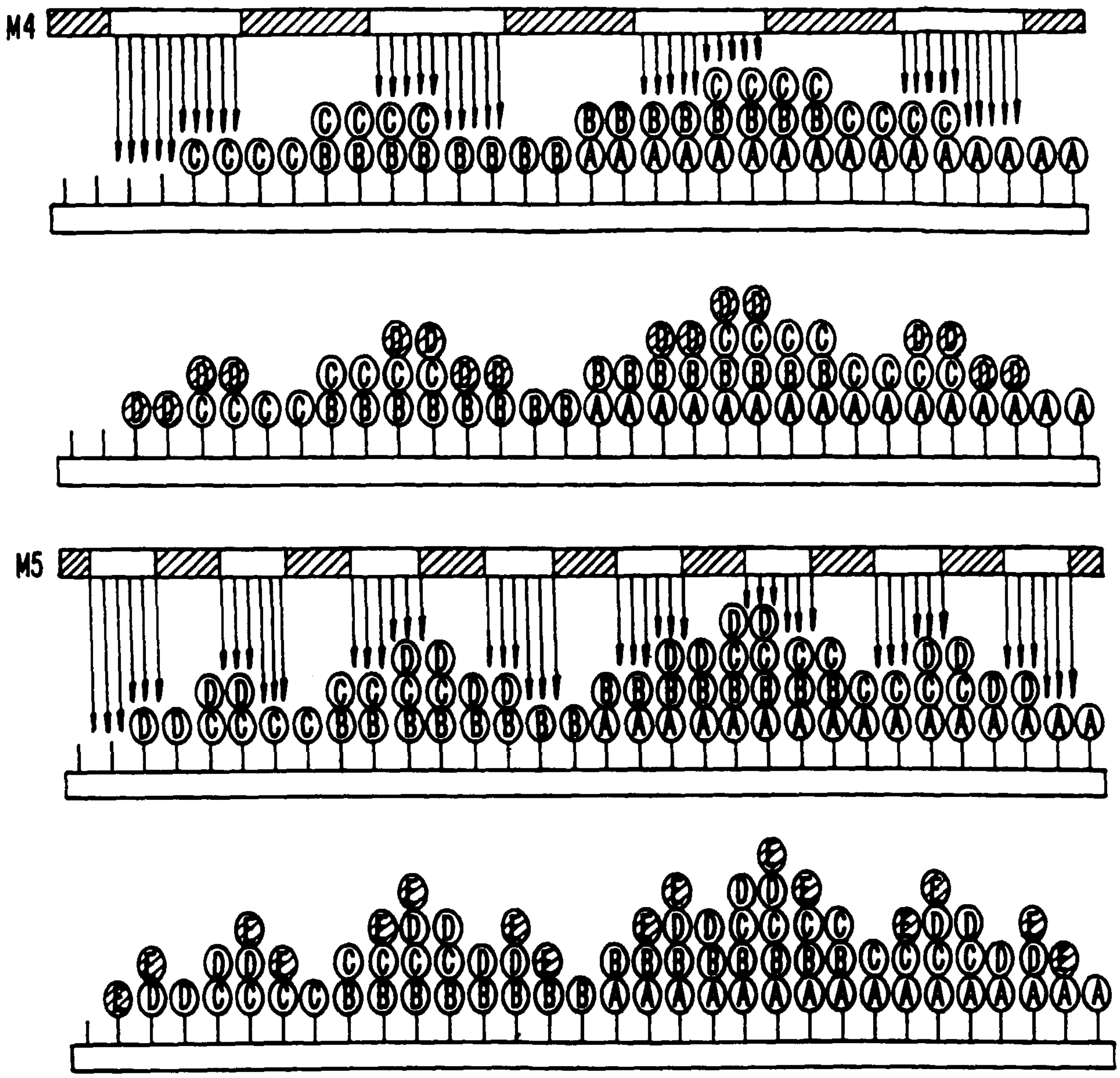


FIG. 6B.

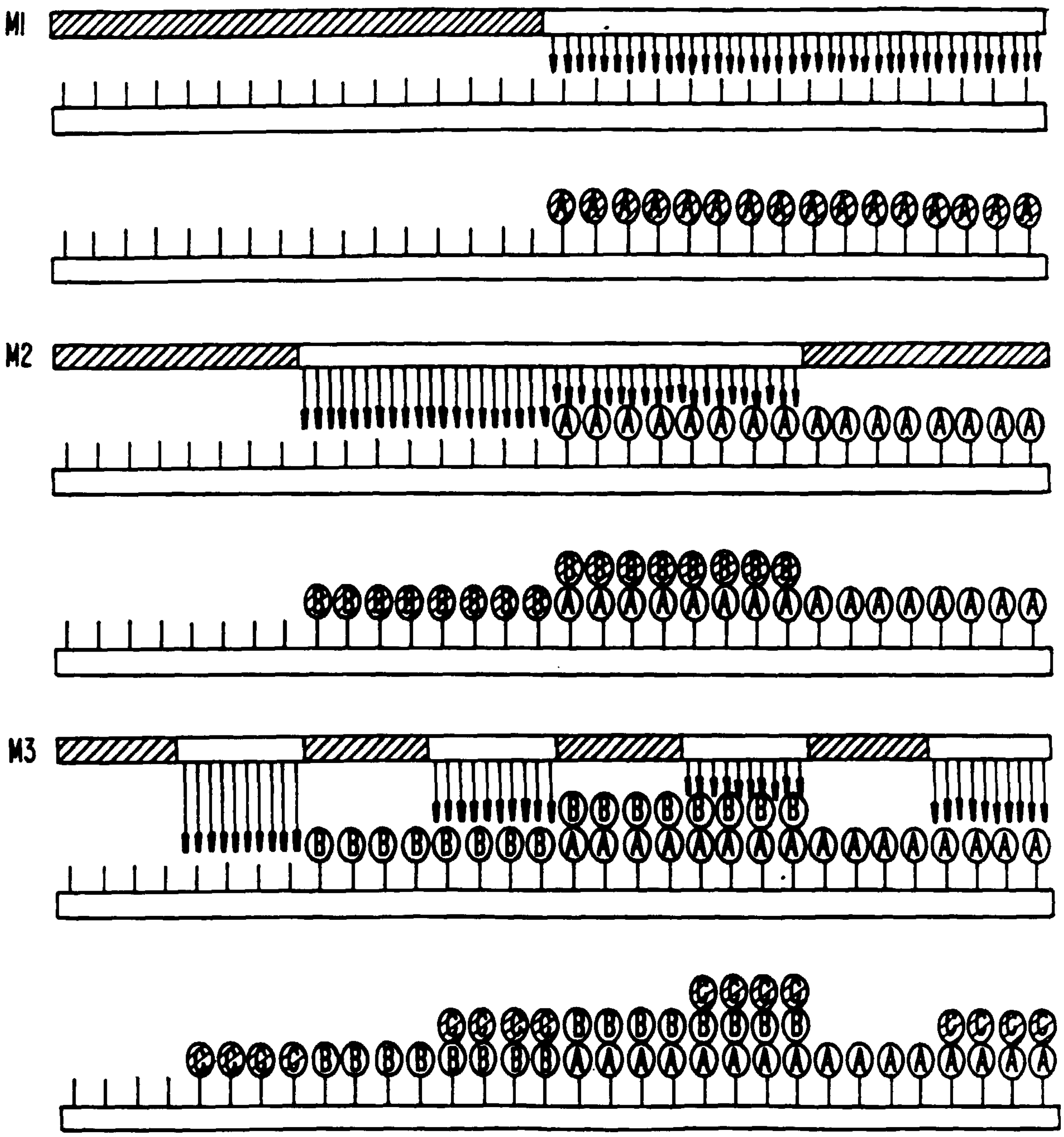


FIG. 7A.

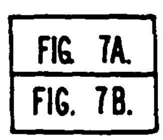


FIG. 7.

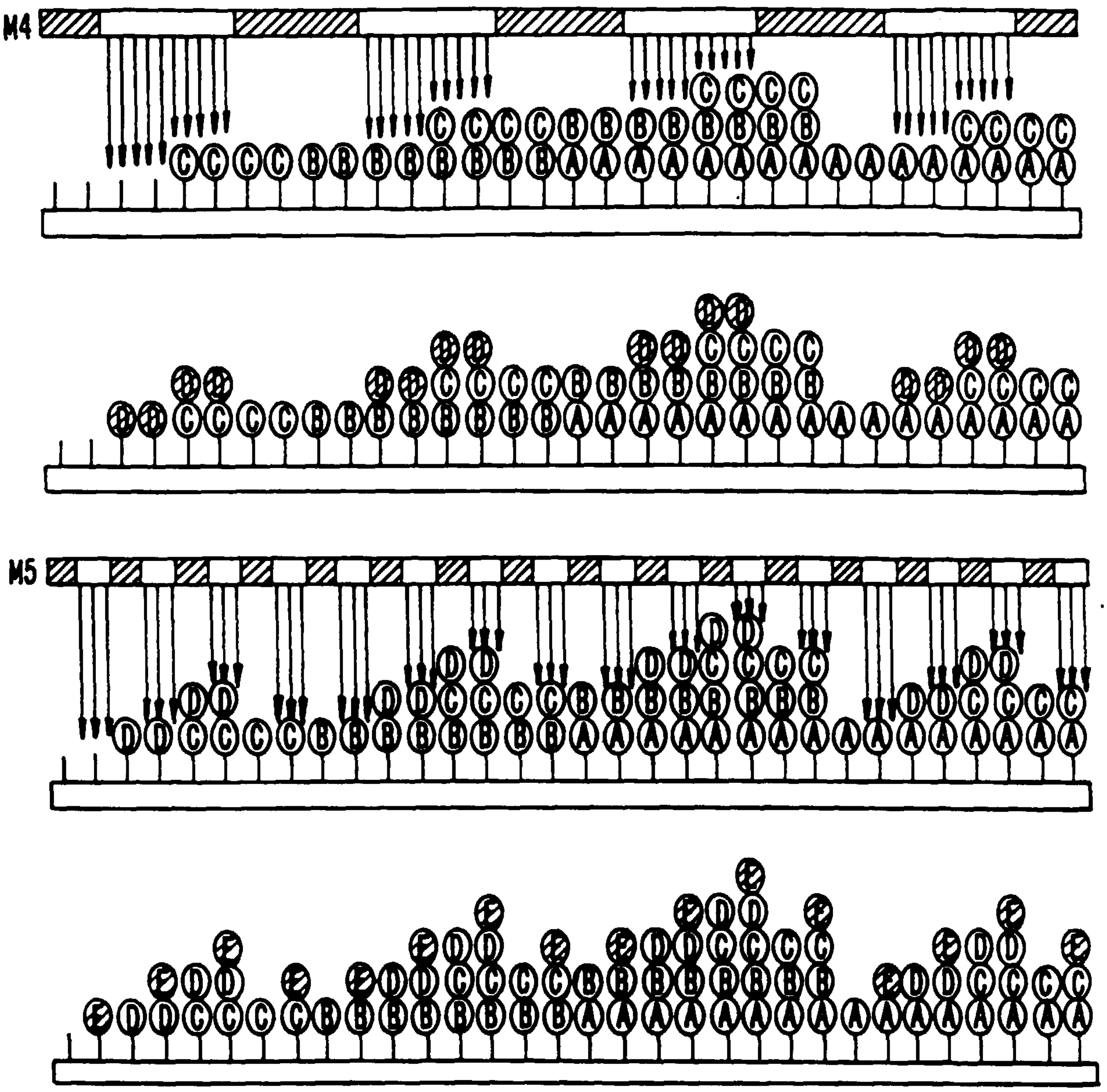


FIG. 7B.

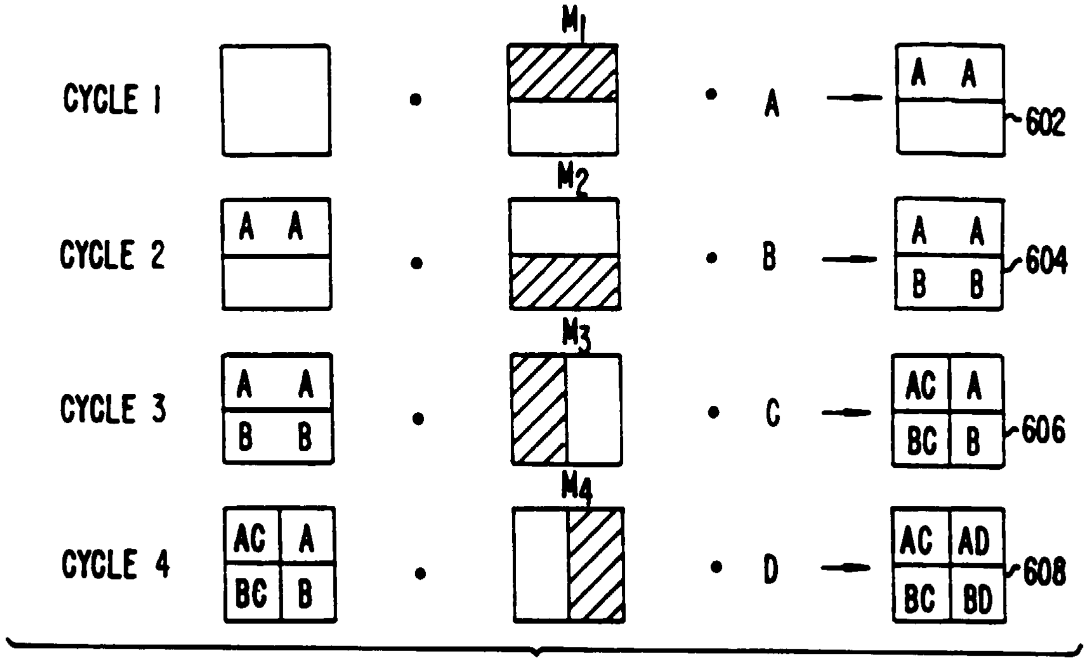

CYCLE 1
M1
A
A A
602
CYCLE 2
M2
B
A A
B B
604
CYCLE 3
M3
C
AC A
BC B
606
CYCLE 4
M4
D
AC AD
BC BD
608


FIG. 8A.

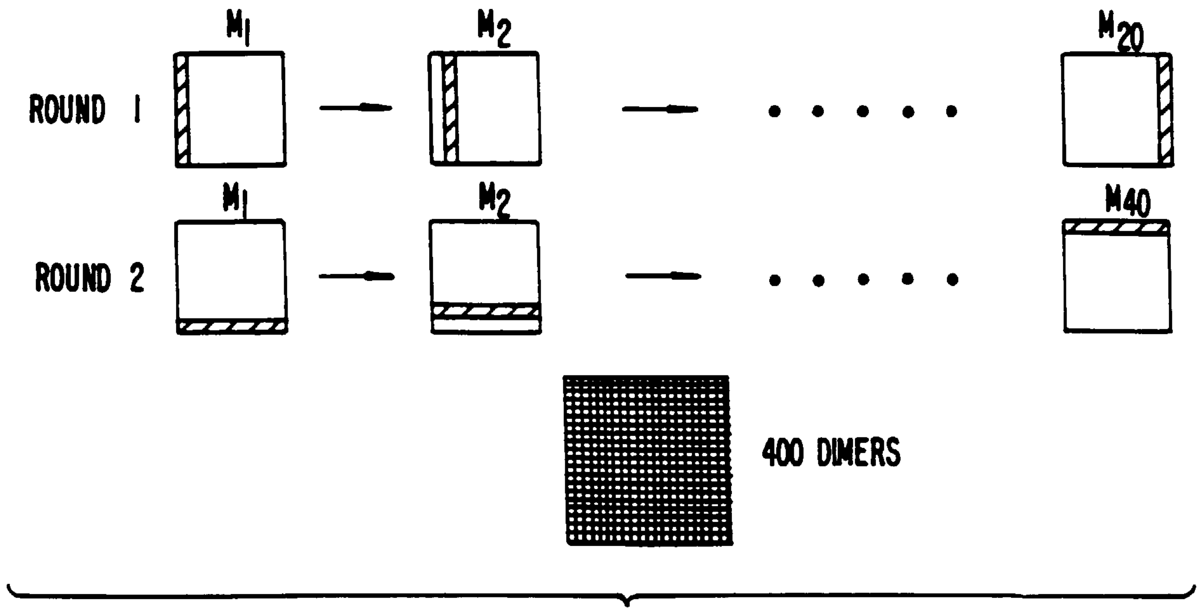

ROUND 1
M1
M2
M20
ROUND 2
M1
M2
M40
400 DIMERS


FIG. 8B.

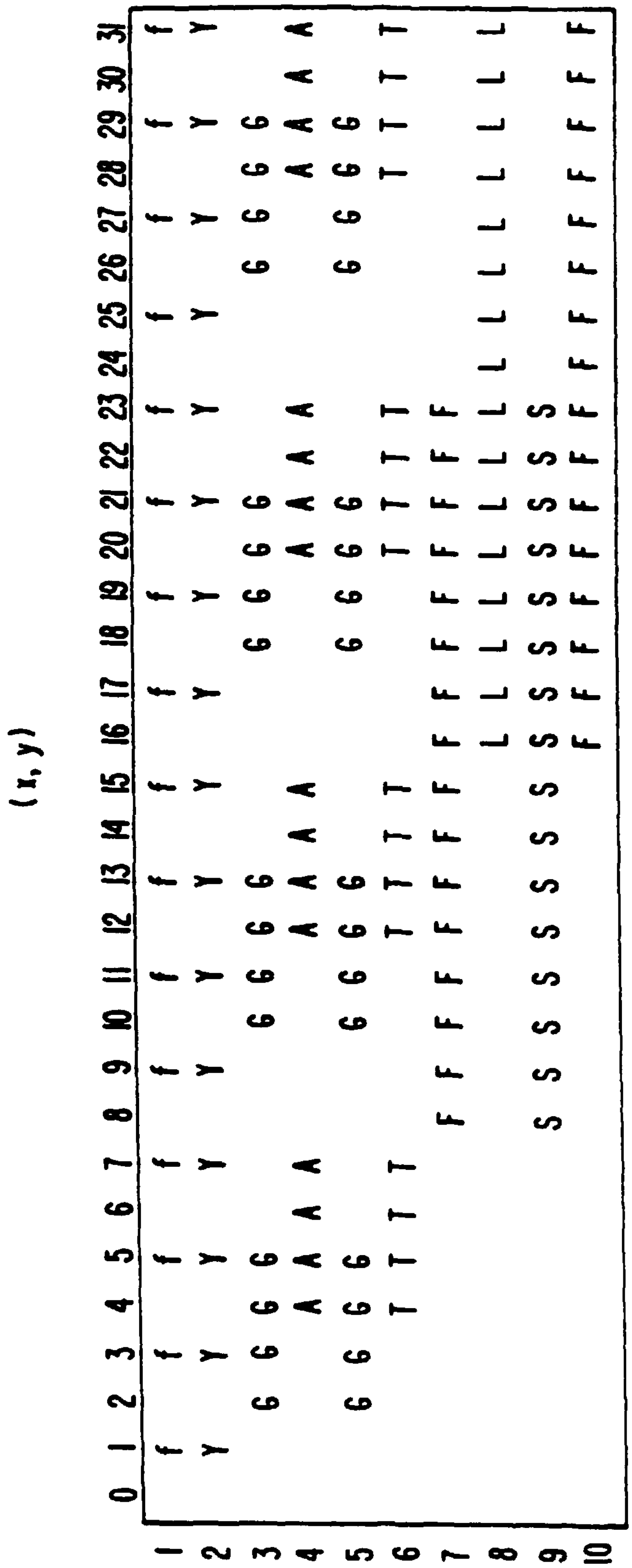


FIG. 9.

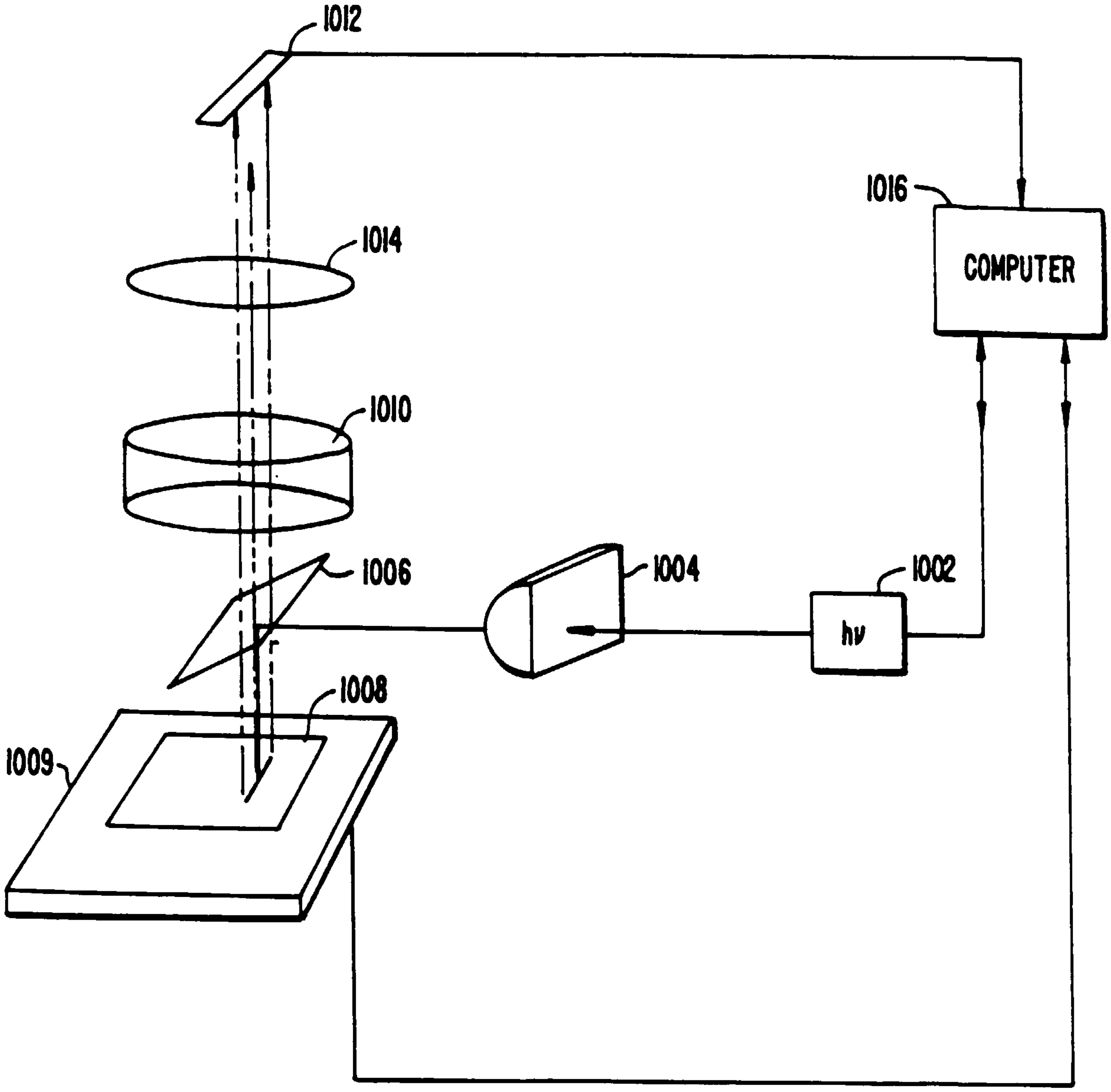


FIG. 10.

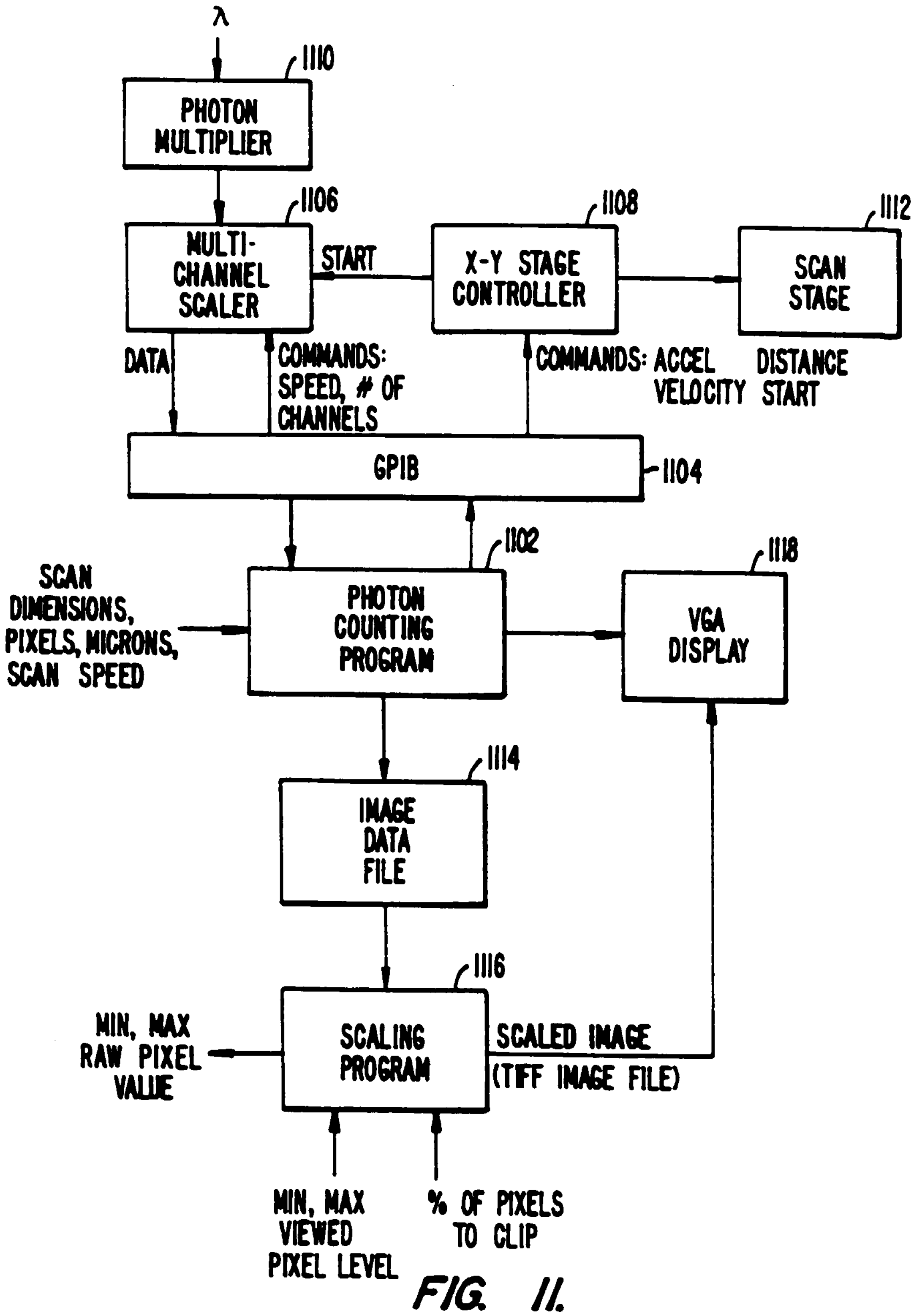
λ
1110
PHOTON MULTIPLIER
1106
MULTI-CHANNEL SCALER
START
1108
X-Y STAGE CONTROLLER
1112
SCAN STAGE
DATA
COMMANDS: SPEED, # OF CHANNELS
COMMANDS: ACCEL VELOCITY DISTANCE START
GPIB
1104
1102
SCAN DIMENSIONS, PIXELS, MICRONS, SCAN SPEED
PHOTON COUNTING PROGRAM
1118
VGA DISPLAY
1114
IMAGE DATA FILE
1116
MIN, MAX RAW PIXEL VALUE
SCALING PROGRAM
SCALED IMAGE (TIFF IMAGE FILE)
MIN, MAX VIEWED PIXEL LEVEL
% OF PIXELS TO CLIP

FIG. 11.

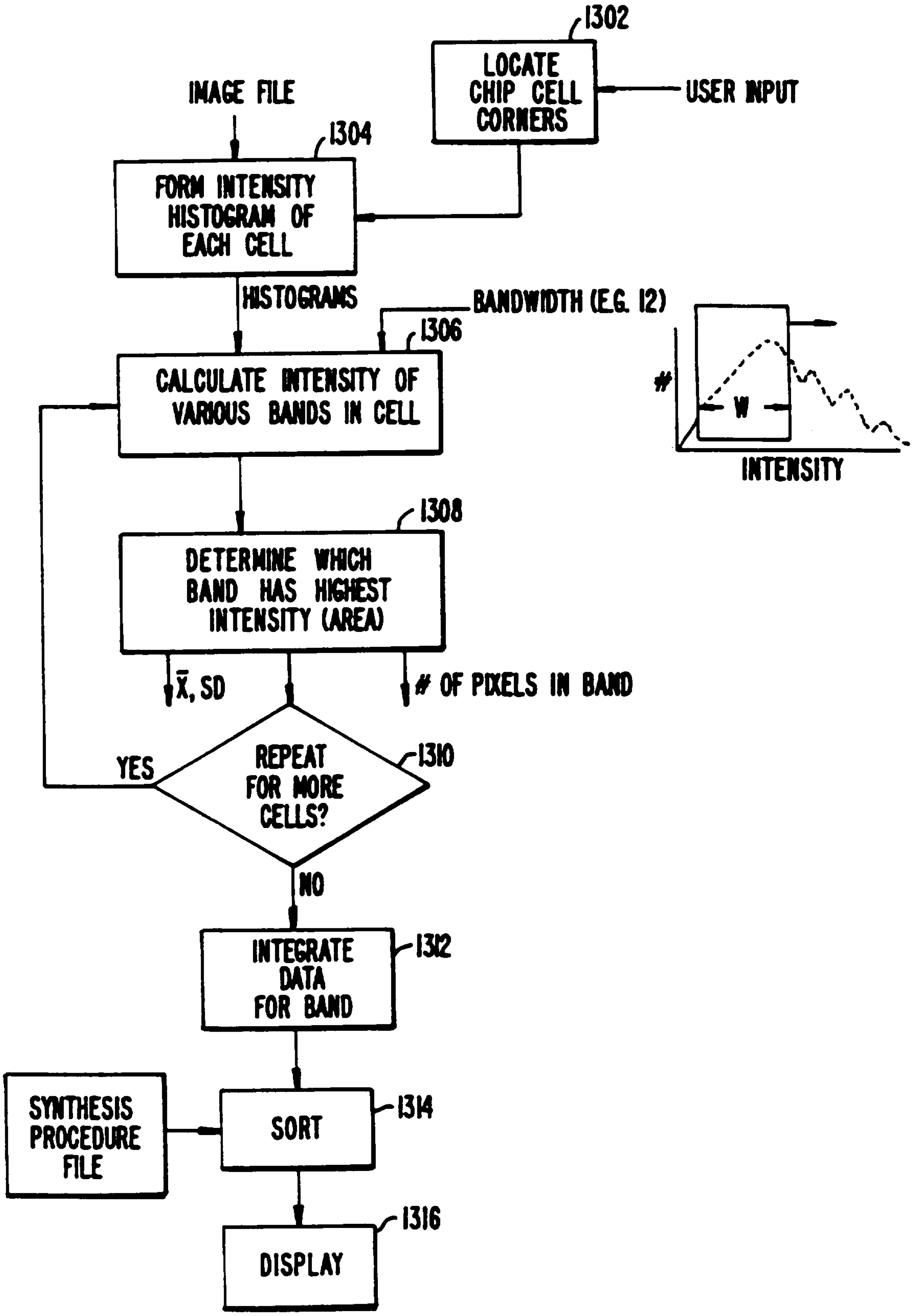
1302
LOCATE CHIP CELL CORNERS
USER INPUT
IMAGE FILE
1304
FORM INTENSITY HISTOGRAM OF EACH CELL
HISTOGRAMS
BANDWIDTH (E.G. 12)
1306
CALCULATE INTENSITY OF VARIOUS BANDS IN CELL
#
W
INTENSITY
1308
DETERMINE WHICH BAND HAS HIGHEST INTENSITY (AREA)
$\bar{X}$, SD
# OF PIXELS IN BAND
YES
REPEAT FOR MORE CELLS?
1310
NO
INTEGRATE DATA FOR BAND
1312
SYNTHESIS PROCEDURE FILE
SORT
1314
DISPLAY
1316

*FIG. 12.*

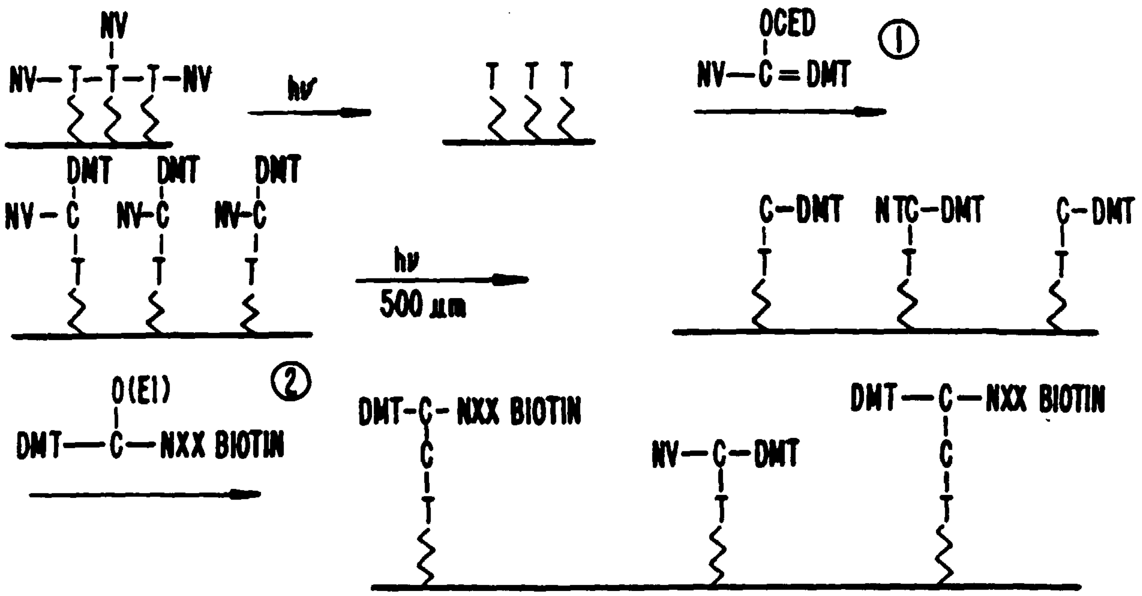


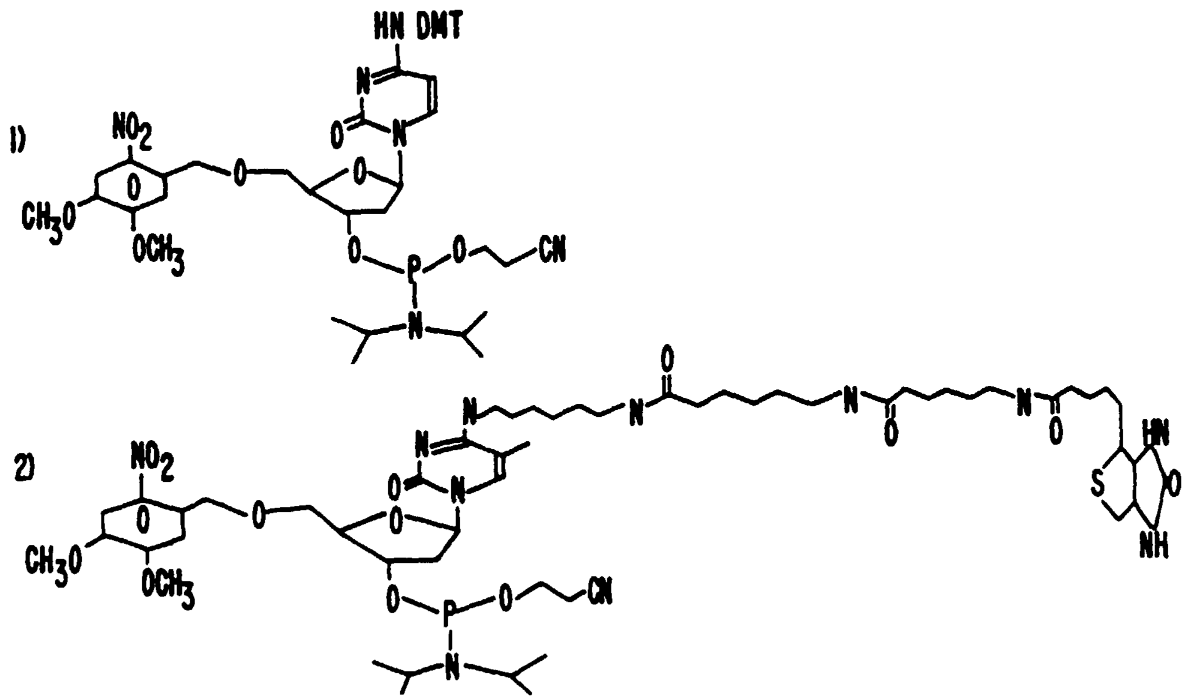
1)
2)

FIG. 13.